**(19)** Europäisches Patentamt

European Patent Office

Office européen des brevets

**(11)** **EP 1 156 117 A2**

**(12)** **EUROPÄISCHE PATENTANMELDUNG**

**(43)** Veröffentlichungstag:
**21.11.2001 Patentblatt 2001/47**

**(51)** Int Cl.7: **C12N 15/82**, C12N 9/02,
C12N 5/10

**(21)** Anmeldenummer: **01109477.8**

**(22)** Anmeldetag: **25.04.2001**

**(84)** Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

**(30)** Priorität: **08.05.2000 DE 10022362**

**(71)** Anmelder: **BAYER AG**
**51368 Leverkusen (DE)**

**(72)** Erfinder:
• **Busch, Marco**
**40764 Langenfeld (DE)**
• **Hain, Rüdiger, Prof. Dr.**
**40764 Langenfeld (DE)**

**(54) Verfahren zum Auffinden von Modulatoren von Enzymen des Carotenoid-Biosyntheseweges**

**(57)** Die Erfindung betrifft Nukleinsäuren, die für die die Zeta Carotene Synthase aus Tabak kodieren, davon kodierte Polypeptide sowie Verfahren zum Auffinden von Modulatoren der Aktivität der Zeta Carotene Synthase, der Phytoene Synthase und der Phytoene Desaturase.

SEQUENZPROTOKOLL

<110> BAYER AG

<120> DNA kodierend für die Phytoene Synthase aus Tabak

<130> Le A 34 326

<140>
<141>

<160> 10

<170> PatentIn Ver. 2.1

<210> 1
<211> 1728
<212> DNA
<213> Nicotiana tabacum

<220>
<221> CDS
<222> (244)..(1566)

<400> 1
agaaacccag aaagaacaac aggttttgct tcttgttgat gagtgcattt gcctctgctt 60

gtgtaaggca aagtcggttc actttcttat atccgatttt tataatcgtt gaaattagtg
120

gatagactct agtggatatc tacaagtatt ggttttttga taaaataggc tgaggtgaga
180

aggtaacata aaggaaagac aaaaacttgg gaattgtttt agaccaccga ggtttcttgt
240

ttc atg agc atg tct gtt gct ttg ttg tgg gtt gtt tct ccc act tcc
288
    Met Ser Met Ser Val Ala Leu Leu Trp Val Val Ser Pro Thr Ser
    1               5                   10                  15

gag gtc tcg aat ggg aca gga ttg ttg gat tca gtc cga gaa gga aac
336
Glu Val Ser Asn Gly Thr Gly Leu Leu Asp Ser Val Arg Glu Gly Asn
            20                  25                  30

cgc gtc ttt gta tca tcc agg ttc cta gct cga gat agg aat ttg atg
384
Arg Val Phe Val Ser Ser Arg Phe Leu Ala Arg Asp Arg Asn Leu Met
            35                  40                  45

tgg aat ggg aga atc aag aaa ggt ggg aga caa agg tgg aat ttt ggc
432
Trp Asn Gly Arg Ile Lys Lys Gly Gly Arg Gln Arg Trp Asn Phe Gly
            50                  55                  60

tct tta att gct gat cca aga tat tca tgc ttg ggt gga tca aga act
480

---

15

```
Ser Leu Ile Ala Asp Pro Arg Tyr Ser Cys Leu Gly Gly Ser Arg Thr
    65                  70              75

gaa aag gga agc act ttc tct gta cag tcc agt ttg gtg gct agc cca
528

Glu Lys Gly Ser Thr Phe Ser Val Gln Ser Ser Leu Val Ala Ser Pro
    80              85              90              95

gct gga gaa atg act gtg tca tca gag aaa aag gtg tat gat gtg gta
576

Ala Gly Glu Met Thr Val Ser Ser Glu Lys Lys Val Tyr Asp Val Val
                100             105             110

tta aag cag gca gct tta gtg aag agg cag ctg aga tct acc gat gat
624.

Leu Lys Gln Ala Ala Leu Val Lys Arg Gln Leu Arg Ser Thr Asp Asp
            115             120             125

tta gaa gtg aag ccg gat att gtt gtt cca ggg aat ttg ggc ttg ttg
672

Leu Glu Val Lys Pro Asp Ile Val Val Pro Gly Asn Leu Gly Leu Leu
        130             135             140

agt gaa gca tat gat cgt tgt ggc gaa gta tgt gca gag tat gca aag
720

Ser Glu Ala Tyr Asp Arg Cys Gly Glu Val Cys Ala Glu Tyr Ala Lys
    145             150             155

aca ttt tac tta gga acc aag cta atg acc cca gag aga aga aga gct
768

Thr Phe Tyr Leu Gly Thr Lys Leu Met Thr Pro Glu Arg Arg Arg Ala
160             165             170             175

atc tgg gca ata tat gtg tgg tgc agg aga acg gat gag ctt gtt gat
816

Ile Trp Ala Ile Tyr Val Trp Cys Arg Arg Thr Asp Glu Leu Val Asp
            180             185             190

ggc cct aat gca tcc cac ata act ccg caa gct tta gat agg tgg gag
864

Gly Pro Asn Ala Ser His Ile Thr Pro Gln Ala Leu Asp Arg Trp Glu
            195             200             205

acc agg ctg gaa gat att ttc agt ggg cgg cca ttt gat atg ctt gat
912

Thr Arg Leu Glu Asp Ile Phe Ser Gly Arg Pro Phe Asp Met Leu Asp
            210             215             220

gct gct tta tcc gat act gtc tcc aga ttt cct gtt gat att cag cca
960

Ala Ala Leu Ser Asp Thr Val Ser Arg Phe Pro Val Asp Ile Gln Pro
    225             230             235
```

ttc aga gat atg att gaa gga atg cgt atg gac ttg tgg aaa tcc aga
1008

Phe Arg Asp Met Ile Glu Gly Met Arg Met Asp Leu Trp Lys Ser Arg
240         245             250             255

tac aaa act ttc gat gag cta tat ctc tat tgt tac tat gtt gct ggt
1056

Tyr Lys Thr Phe Asp Glu Leu Tyr Leu Tyr Cys Tyr Tyr Val Ala Gly
            260             265             270

act gta gga ttg atg agt gtt cca gtt atg ggt att gca cct gaa tca
1104

Thr Val Gly Leu Met Ser Val Pro Val Met Gly Ile Ala Pro Glu Ser
            275             280             285

aag gca aca aca gag agt gta tat aat gct gct ttg gct tta ggg ctt
1152

Lys Ala Thr Thr Glu Ser Val Tyr Asn Ala Ala Leu Ala Leu Gly Leu
            290             295             300

gca aat caa cta acc aat ata ctc aga gat gta gga gaa gat gcc aga
1200

Ala Asn Gln Leu Thr Asn Ile Leu Arg Asp Val Gly Glu Asp Ala Arg
        305             310             315

aga gga aga gta tac ttg cct caa gat gaa tta gca cag gca ggg ctc
1248

Arg Gly Arg Val Tyr Leu Pro Gln Asp Glu Leu Ala Gln Ala Gly Leu
320             325             330             335

tcc gac gaa gac ata ttt gct gga aga gtg act gat aag tgg agg aac
1296

Ser Asp Glu Asp Ile Phe Ala Gly Arg Val Thr Asp Lys Trp Arg Asn
            340             345             350

ttt atg aag aaa caa att cag agg gcg agg aaa ttc ttt gat gag tca
1344

Phe Met Lys Lys Gln Ile Gln Arg Ala Arg Lys Phe Phe Asp Glu Ser
            355             360             365

gag aaa ggt gtc aca gaa ctg gac tct gct agt aga tgg cct gtg tta
1392

Glu Lys Gly Val Thr Glu Leu Asp Ser Ala Ser Arg Trp Pro Val Leu
            370             375             380

aca gcg ctg ctg ttg tat cgc aag ata ttg gac gag att gaa gcc aac
1440

Thr Ala Leu Leu Leu Tyr Arg Lys Ile Leu Asp Glu Ile Glu Ala Asn
        385             390             395

gac tac aac aac ttc aca agg agg gct tat gtt agc aag cca aag aag
1488

```
Asp Tyr Asn Asn Phe Thr Arg Arg Ala Tyr Val Ser Lys Pro Lys Lys
400             405             410             415

ctt ctc acc ttg ccc att gct tat gca aaa tct ctt gtg ccc cct aat
1536

Leu Leu Thr Leu Pro Ile Ala Tyr Ala Lys Ser Leu Val Pro Pro Asn
            420             425             430

aga act tcc tct cca cta gca aag aca tga atgaagtagt tgagtcaatg
1586

Arg Thr Ser Ser Pro Leu Ala Lys Thr
        435             440

agtattatac actaaagaaa ctcaggtact tgtaaatgag atatcttttg ctaaatgtgt
1646

atcatcaaaa gtagattgta aattcaatat gacaatctct tggtagaata ttttctccac
1706

actcatcaaa                    ccctcaagtg                    ag
1728
```

`<210>` 2
`<211>` 440
`<212>` PRT
`<213>` Nicotiana tabacum

`<400>` 2
```
Met Ser Met Ser Val Ala Leu Leu Trp Val Val Ser Pro Thr Ser Glu
 1               5               10              15

Val Ser Asn Gly Thr Gly Leu Leu Asp Ser Val Arg Glu Gly Asn Arg
            20              25              30

Val Phe Val Ser Ser Arg Phe Leu Ala Arg Asp Arg Asn Leu Met Trp
        35              40              45

Asn Gly Arg Ile Lys Lys Gly Gly Arg Gln Arg Trp Asn Phe Gly Ser
    50              55              60

Leu Ile Ala Asp Pro Arg Tyr Ser Cys Leu Gly Gly Ser Arg Thr Glu
65              70              75              80

Lys Gly Ser Thr Phe Ser Val Gln Ser Ser Leu Val Ala Ser Pro Ala
            85              90              95

Gly Glu Met Thr Val Ser Ser Glu Lys Lys Val Tyr Asp Val Val Leu
            100             105             110

Lys Gln Ala Ala Leu Val Lys Arg Gln Leu Arg Ser Thr Asp Asp Leu
        115             120             125

Glu Val Lys Pro Asp Ile Val Val Pro Gly Asn Leu Gly Leu Leu Ser
        130             135             140

Glu Ala Tyr Asp Arg Cys Gly Glu Val Cys Ala Glu Tyr Ala Lys Thr
145             150             155             160

Phe Tyr Leu Gly Thr Lys Leu Met Thr Pro Glu Arg Arg Arg Ala Ile
                165             170             175
```

```
Trp Ala Ile Tyr Val Trp Cys Arg Arg Thr Asp Glu Leu Val Asp Gly
            180                 185                 190

Pro Asn Ala Ser His Ile Thr Pro Gln Ala Leu Asp Arg Trp Glu Thr
            195                 200                 205

Arg Leu Glu Asp Ile Phe Ser Gly Arg Pro Phe Asp Met Leu Asp Ala
            210                 215                 220

Ala Leu Ser Asp Thr Val Ser Arg Phe Pro Val Asp Ile Gln Pro Phe
225                 230                 235                 240

Arg Asp Met Ile Glu Gly Met Arg Met Asp Leu Trp Lys Ser Arg Tyr
                245                 250                 255

Lys Thr Phe Asp Glu Leu Tyr Leu Tyr Cys Tyr Tyr Val Ala Gly Thr
            260                 265                 270

Val Gly Leu Met Ser Val Pro Val Met Gly Ile Ala Pro Glu Ser Lys
            275                 280                 285

Ala Thr Thr Glu Ser Val Tyr Asn Ala Ala Leu Ala Leu Gly Leu Ala
            290                 295                 300

Asn Gln Leu Thr Asn Ile Leu Arg Asp Val Gly Glu Asp Ala Arg Arg
305                 310                 315                 320

Gly Arg Val Tyr Leu Pro Gln Asp Glu Leu Ala Gln Ala Gly Leu Ser
                325                 330                 335

Asp Glu Asp Ile Phe Ala Gly Arg Val Thr Asp Lys Trp Arg Asn Phe
                340                 345                 350

Met Lys Lys Gln Ile Gln Arg Ala Arg Lys Phe Phe Asp Glu Ser Glu
            355                 360                 365

Lys Gly Val Thr Glu Leu Asp Ser Ala Ser Arg Trp Pro Val Leu Thr
            370                 375                 380

Ala Leu Leu Leu Tyr Arg Lys Ile Leu Asp Glu Ile Glu Ala Asn Asp
385                 390                 395                 400

Tyr Asn Asn Phe Thr Arg Arg Ala Tyr Val Ser Lys Pro Lys Lys Leu
                405                 410                 415

Leu Thr Leu Pro Ile Ala Tyr Ala Lys Ser Leu Val Pro Pro Asn Arg
                420                 425                 430

Thr Ser Ser Pro Leu Ala Lys Thr
            435                 440


<210> 3
<211> 1712
<212> DNA
<213> Nicotiana tabacum

<220>
<221> CDS
<222> (333)..(1565)
```

```
<400> 3
cttgaagagt agcagcagca agcaagahaa ttaaagtggg ctatttbkka naagccattg 60

ttacmagara attaagaagc caagamacag gttattttct acttgagtya ggaaaagttg
120

gtttgcttta tttgtgggct ttttataatc tttttccac aagggaaagt gggtattttc
180

ttgaaagtgg atttagactc tagtgggaat ctactaggag taaatttatt aattttttat
240

aaattaagca gaggaaggaa ggaaacagaa aacagaaagt aagacaaaaa accttggaat
300

tgttttagaa agccaaggtt ttcctgttca aa atg tct gtt gcc ttg tta tgg
353.
                                      Met Ser Val Ala Leu Leu Trp
                                       1               5

gtt gtt tca cct tgt gaa gtc tca aat ggg aca gga ttc ttg gat tca
401
Val Val Ser Pro Cys Glu Val Ser Asn Gly Thr Gly Phe Leu Asp Ser
          10              15              20

gtc cgg gag gga aac cgg gtt ttt gat tcg tcg agg cat agg aat tta
449
Val Arg Glu Gly Asn Arg Val Phe Asp Ser Ser Arg His Arg Asn Leu
      25              30              35

gtg tgc aat gag aga aac aag aga ggt gtg aaa caa agg tgg aat ttt
497
Val Cys Asn Glu Arg Asn Lys Arg Gly Val Lys Gln Arg Trp Asn Phe
   40              45              50              55

ggt tct gta agg tct gct atg gtg gct aca ccg gcg gga gaa atg gcg
545
Gly Ser Val Arg Ser Ala Met Val Ala Thr Pro Ala Gly Glu Met Ala
          60              65              70

acg atg aca tca gaa cag atg gtt tat gat gtg gtt tta aaa caa gca
593
Thr Met Thr Ser Glu Gln Met Val Tyr Asp Val Val Leu Lys Gln Ala
          75              80              85

gct tta gtg aag agg cag ttg aga tct gct gat gat tta gaa gtg aag
641
Ala Leu Val Lys Arg Gln Leu Arg Ser Ala Asp Asp Leu Glu Val Lys
          90              95              100

ccg gag atc cct ctc ccc ggg aat ttg agc ttg ttg agt gaa gca tat
689
Pro Glu Ile Pro Leu Pro Gly Asn Leu Ser Leu Leu Ser Glu Ala Tyr
          105             110             115
```

```
gat agg tgt agt gaa gta tgt gca gag tat gca aag aca ttt tac tth
737

Asp Arg Cys Ser Glu Val Cys Ala Glu Tyr Ala Lys Thr Phe Tyr Xaa
120             125             130             135

gga acc atg yta atg act cca gag aga aga agg gct att tgg gca ata
785

Gly Thr Met Xaa Met Thr Pro Glu Arg Arg Arg Ala Ile Trp Ala Ile
            140             145             150

tat gtg tgg tgc agg aga aca gat gaa ctt gtt gat ggc cca aac gca
833

Tyr Val Trp Cys Arg Arg Thr Asp Glu Leu Val Asp Gly Pro Asn Ala
            155             160             165

tca cat att aca ccc caa gcc tta gat agg tgg gaa gac cgg ctt gaa
881

Ser His Ile Thr Pro Gln Ala Leu Asp Arg Trp Glu Asp Arg Leu Glu
            170             175             180

gat gtt ttc agc ggg cga cca ttt gat atg ctc gat gct gct ttg tcc
929

Asp Val Phe Ser Gly Arg Pro Phe Asp Met Leu Asp Ala Ala Leu Ser
            185             190             195

gat act gtt tcc aag ttt cca gtt gat att cag ccg ttc aga gat atg
977

Asp Thr Val Ser Lys Phe Pro Val Asp Ile Gln Pro Phe Arg Asp Met
200             205             210             215

att gaa gga atg cgt atg gac ttg agg aag tca aga tat aga aac ttt
1025

Ile Glu Gly Met Arg Met Asp Leu Arg Lys Ser Arg Tyr Arg Asn Phe
            220             225             230

gat gag ctt tac ctc tat tgt tat tac gtt gct ggt acg gtt ggg ttg
1073

Asp Glu Leu Tyr Leu Tyr Cys Tyr Tyr Val Ala Gly Thr Val Gly Leu
            235             240             245

atg agt gtt cca att atg ggt att gca cct gat tca aag gca aca aca
1121

Met Ser Val Pro Ile Met Gly Ile Ala Pro Asp Ser Lys Ala Thr Thr
            250             255             260

gag agc gta tat aat gca gct ttg gct tta gga atc gca aat caa cta
1169

Glu Ser Val Tyr Asn Ala Ala Leu Ala Leu Gly Ile Ala Asn Gln Leu
            265             270             275

acg aac ata ctc aga gat gtt gga gaa gat gcc aga aga gga aga gtc
1217
```

```
Thr Asn Ile Leu Arg Asp Val Gly Glu Asp Ala Arg Arg Gly Arg Val
280                 285                 290                 295

tac tta cct caa gat gaa tta gca cag gca ggt ctc ttc gac gat gac
1265

Tyr Leu Pro Gln Asp Glu Leu Ala Gln Ala Gly Leu Phe Asp Asp Asp
                300                 305                 310

ata ttt gct gga aaa gtg act gat aag tgg aga agc ttt atg aag aag
1313

Ile Phe Ala Gly Lys Val Thr Asp Lys Trp Arg Ser Phe Met Lys Lys
            315                 320                 325

caa atc cag agg gca aga aag ttc ttc gat gag gca gag gaa gga gtt
1361

Gln Ile Gln Arg Ala Arg Lys Phe Phe Asp Glu Ala Glu Glu Gly Val
            330                 335                 340

aca caa ctg agc tca gct agc aga tgg cct gta tgg gca tct ttg ctg
1409

Thr Gln Leu Ser Ser Ala Ser Arg Trp Pro Val Trp Ala Ser Leu Leu
            345                 350                 355

ttg tac cgc caa ata ctg gac gag att gaa gcc aat gac tac aac aac
1457

Leu Tyr Arg Gln Ile Leu Asp Glu Ile Glu Ala Asn Asp Tyr Asn Asn
360                 365                 370                 375

ttc aca aag aga gct tat gtg agc aaa cca aag aag cta att tcc tta
1505

Phe Thr Lys Arg Ala Tyr Val Ser Lys Pro Lys Lys Leu Ile Ser Leu
            380                 385                 390

cct att gct tat gca aaa tct ctt gtg ccc cct aca aga act ctt gtc
1553

Pro Ile Ala Tyr Ala Lys Ser Leu Val Pro Pro Thr Arg Thr Leu Val
            395                 400                 405

acc tct agc taa ggcatagaca tcagatttaa attaaagcaa gaaagcatat
1605

Thr Ser Ser
            410

actgttaaaa aagaaagaat ttctaaagta gatattgttg tattgatgcc acttgtatat
1665

catcaaaagt    aggtagtaaa    atccaatata    acaatctcta    gtagttg
1712


<210> 4
<211> 410
<212> PRT
<213> Nicotiana tabacum
```

```
<400> 4
Met Ser Val Ala Leu Leu Trp Val Val Ser Pro Cys Glu Val Ser Asn
 1               5                  10                  15

Gly Thr Gly Phe Leu Asp Ser Val Arg Glu Gly Asn Arg Val Phe Asp
            20                  25                  30

Ser Ser Arg His Arg Asn Leu Val Cys Asn Glu Arg Asn Lys Arg Gly
        35                  40                  45

Val Lys Gln Arg Trp Asn Phe Gly Ser Val Arg Ser Ala Met Val Ala
    50                  55                  60

Thr Pro Ala Gly Glu Met Ala Thr Met Thr Ser Glu Gln Met Val Tyr
65                  70                  75                  80

Asp Val Val Leu Lys Gln Ala Ala Leu Val Lys Arg Gln Leu Arg Ser
                85                  90                  95

Ala Asp Asp Leu Glu Val Lys Pro Glu Ile Pro Leu Pro Gly Asn Leu
            100                 105                 110

Ser Leu Leu Ser Glu Ala Tyr Asp Arg Cys Ser Glu Val Cys Ala Glu
        115                 120                 125

Tyr Ala Lys Thr Phe Tyr Xaa Gly Thr Met Xaa Met Thr Pro Glu Arg
    130                 135                 140

Arg Arg Ala Ile Trp Ala Ile Tyr Val Trp Cys Arg Arg Thr Asp Glu
145                 150                 155                 160

Leu Val Asp Gly Pro Asn Ala Ser His Ile Thr Pro Gln Ala Leu Asp
                165                 170                 175

Arg Trp Glu Asp Arg Leu Glu Asp Val Phe Ser Gly Arg Pro Phe Asp
            180                 185                 190

Met Leu Asp Ala Ala Leu Ser Asp Thr Val Ser Lys Phe Pro Val Asp
        195                 200                 205

Ile Gln Pro Phe Arg Asp Met Ile Glu Gly Met Arg Met Asp Leu Arg
    210                 215                 220

Lys Ser Arg Tyr Arg Asn Phe Asp Glu Leu Tyr Leu Tyr Cys Tyr Tyr
225                 230                 235                 240

Val Ala Gly Thr Val Gly Leu Met Ser Val Pro Ile Met Gly Ile Ala
                245                 250                 255

Pro Asp Ser Lys Ala Thr Thr Glu Ser Val Tyr Asn Ala Ala Leu Ala
            260                 265                 270

Leu Gly Ile Ala Asn Gln Leu Thr Asn Ile Leu Arg Asp Val Gly Glu
        275                 280                 285

Asp Ala Arg Arg Gly Arg Val Tyr Leu Pro Gln Asp Glu Leu Ala Gln
    290                 295                 300

Ala Gly Leu Phe Asp Asp Asp Ile Phe Ala Gly Lys Val Thr Asp Lys
305                 310                 315                 320
```

```
Trp Arg Ser Phe Met Lys Lys Gln Ile Gln Arg Ala Arg Lys Phe Phe
                325             330                 335

Asp Glu Ala Glu Glu Gly Val Thr Gln Leu Ser Ser Ala Ser Arg Trp
                340             345                 350

Pro Val Trp Ala Ser Leu Leu Leu Tyr Arg Gln Ile Leu Asp Glu Ile
            355             360                 365

Glu Ala Asn Asp Tyr Asn Asn Phe Thr Lys Arg Ala Tyr Val Ser Lys
        370             375             380

Pro Lys Lys Leu Ile Ser Leu Pro Ile Ala Tyr Ala Lys Ser Leu Val
385             390             395                 400

Pro Pro Thr Arg Thr Leu Val Thr Ser Ser
                405             410
```

```
<210> 5
<211> 2205
<212> DNA
<213> Nicotiana tabacum

<220>
<221> CDS
<222> (189)..(1955)

<400> 5
ctggcatctt acatctgcca aatttctcat ttatagcatc tcctaatctt tagatacctt 60

ttcttcttgt tttgttttc tatccttcac ttcatgcttt cttgttttac ccatctcttc
120

cattttcttg gcatttgaca acaaaaggtt ccatttttt tccttttgc tgtatatagc
180

acaattca atg gct act tct tca gct tat ctt tgt tgt cct gca act tct
230
        Met Ala Thr Ser Ser Ala Tyr Leu Cys Cys Pro Ala Thr Ser
            1               5                   10

gct act gga aag aaa cat att ttg cca aat ggg tca gct gga ttc ttg
278
Ala Thr Gly Lys Lys His Ile Leu Pro Asn Gly Ser Ala Gly Phe Leu
 15                 20              25                  30

gtt ttc cgt ggt ccc cgt ttg tcc aac cgg ttt gtg acc cgg aag tca
326
Val Phe Arg Gly Pro Arg Leu Ser Asn Arg Phe Val Thr Arg Lys Ser
                35              40                  45

gtt att cgt gct gat ttg gac tcc atg gtc tct gat atg agt act aat
374
Val Ile Arg Ala Asp Leu Asp Ser Met Val Ser Asp Met Ser Thr Asn
            50              55                  60

gct cca aaa ggg cta ttt cca cct gaa cct gaa cat tat cgg ggg cca
422
```

```
Ala Pro Lys Gly Leu Phe Pro Pro Glu Pro Glu His Tyr Arg Gly Pro
        65                  70                  75

aag ctg aaa gta gct att att gga gct ggg ctt gca ggc atg tca act
470

Lys Leu Lys Val Ala Ile Ile Gly Ala Gly Leu Ala Gly Met Ser Thr
        80                  85                  90

gct gtg gag ctc ttg gat caa gga cat gag gtg gat ata tat gaa tca
518

Ala Val Glu Leu Leu Asp Gln Gly His Glu Val Asp Ile Tyr Glu Ser
    95                  100                 105                 110

agg cct ttt att ggt ggg aaa gtg gga tct ttt gtt gat aga cgt gga
566.

Arg Pro Phe Ile Gly Gly Lys Val Gly Ser Phe Val Asp Arg Arg Gly
                115                 120                 125

aac cac att gaa atg gga ctg cat gtg ttc ttt ggt tgc tat aat aat
614

Asn His Ile Glu Met Gly Leu His Val Phe Phe Gly Cys Tyr Asn Asn
                130                 135                 140

ttg ttc cgt ttg tta aaa aag gtg ggt gct gaa aaa aat ctg cta gtg
662

Leu Phe Arg Leu Leu Lys Lys Val Gly Ala Glu Lys Asn Leu Leu Val
        145                 150                 155

aag gac cat act cac aca ttt gta aat aaa ggg ggt gaa ata ggg gag
710

Lys Asp His Thr His Thr Phe Val Asn Lys Gly Gly Glu Ile Gly Glu
        160                 165                 170

ctt gat ttc cgc ttt cca gtt gga gca ccc cta cac gga att aat gca
758

Leu Asp Phe Arg Phe Pro Val Gly Ala Pro Leu His Gly Ile Asn Ala
175                 180                 185                 190

ttt ttg tct acc aat cag cta aag att tat gat aag gct aga aat gct
806

Phe Leu Ser Thr Asn Gln Leu Lys Ile Tyr Asp Lys Ala Arg Asn Ala
                195                 200                 205

gta gct ctt gcc ctt agt cca gtg gtg cgg gct tta gtt gat cca gat
854

Val Ala Leu Ala Leu Ser Pro Val Val Arg Ala Leu Val Asp Pro Asp
                210                 215                 220

ggc gcg ttg cag cag ata cgt gat cta gat agt gta agc ttt tca gag
902

Gly Ala Leu Gln Gln Ile Arg Asp Leu Asp Ser Val Ser Phe Ser Glu
        225                 230                 235
```

tgg ttt atg tct aaa ggt ggg acg cgt gct agc atc cag agg atg tgg
950

Trp Phe Met Ser Lys Gly Gly Thr Arg Ala Ser Ile Gln Arg Met Trp
240 245 250

gat cct gtc gca tat gct ctt gga ttc att gac tgt gac aat atc agt
998

Asp Pro Val Ala Tyr Ala Leu Gly Phe Ile Asp Cys Asp Asn Ile Ser
255 260 265 270

gct cgg tgt atg ctc act ata ttt gca tta ttt gcc act aaa acg gag
1046

Ala Arg Cys Met Leu Thr Ile Phe Ala Leu Phe Ala Thr Lys Thr Glu
275 280 285

gct tcc cta tta cgc atg ctt aaa ggt tct ccg gac gtt tat ttg agt
1094

Ala Ser Leu Leu Arg Met Leu Lys Gly Ser Pro Asp Val Tyr Leu Ser
290 295 300

ggt cca att aag aag tac atc ttg gat aag ggg gga agg ttt cac atg
1142

Gly Pro Ile Lys Lys Tyr Ile Leu Asp Lys Gly Gly Arg Phe His Met
305 310 315

agg tgg ggg tgc aga cag gta ctc tat gag aca tcc tct gat ggc agt
1190

Arg Trp Gly Cys Arg Gln Val Leu Tyr Glu Thr Ser Ser Asp Gly Ser
320 325 330

atg tat gtc agc ggg ctt gcc atg tca aag gcc act cag aag aaa gtt
1238

Met Tyr Val Ser Gly Leu Ala Met Ser Lys Ala Thr Gln Lys Lys Val
335 340 345 350

gta aaa gct gat gcc tat gtc gct gca tgt gat gtc cct gga att aaa
1286

Val Lys Ala Asp Ala Tyr Val Ala Ala Cys Asp Val Pro Gly Ile Lys
355 360 365

cga ttg gta cct cag aag tgg agg gaa ttg gaa ttc ttt gac aac att
1334

Arg Leu Val Pro Gln Lys Trp Arg Glu Leu Glu Phe Phe Asp Asn Ile
370 375 380

tac aaa ttg gtt gga gtg cct gtt gtt acg gta caa cta cga tac aat
1382

Tyr Lys Leu Val Gly Val Pro Val Val Thr Val Gln Leu Arg Tyr Asn
385 390 395

ggc tgg gtt aca gag ttg cag gac ttg gag cgt tcg agg caa ttg aag
1430

```
Gly Trp Val Thr Glu Leu Gln Asp Leu Glu Arg Ser Arg Gln Leu Lys
    400             405             410

cgc gct aca ggt ttg gac aat ctc ctg tat aca cca gat gca gat ttc
1478

Arg Ala Thr Gly Leu Asp Asn Leu Leu Tyr Thr Pro Asp Ala Asp Phe
415             420             425             430

tct tgc ttt gcg gac ctt gca ttg gca tct cct gaa gat tat tac att
1526

Ser Cys Phe Ala Asp Leu Ala Leu Ala Ser Pro Glu Asp Tyr Tyr Ile
              435             440             445

gag ggc caa ggc tca ttg ctt caa tgt gtc ctt aca cct ggt gac cct
1574

Glu Gly Gln Gly Ser Leu Leu Gln Cys Val Leu Thr Pro Gly Asp Pro
              450             455             460

tac atg cct cta cta aat gat gaa atc ata aaa aga gtg tca aag cag
1622

Tyr Met Pro Leu Leu Asn Asp Glu Ile Ile Lys Arg Val Ser Lys Gln
          465             470             475

gtt ttg gca cta ttt cct tct tcc caa ggt ctt gag gtt acc tgg tca
1670

Val Leu Ala Leu Phe Pro Ser Ser Gln Gly Leu Glu Val Thr Trp Ser
    480             485             490

tca gtt gtg aaa att ggg caa tcc cta tat cgt gaa gga cct ggt aaa
1718

Ser Val Val Lys Ile Gly Gln Ser Leu Tyr Arg Glu Gly Pro Gly Lys
495             500             505             510

gac cca ttc aga cct gat cag aag act cca gtg gaa aat ttc ttt ctt
1766

Asp Pro Phe Arg Pro Asp Gln Lys Thr Pro Val Glu Asn Phe Phe Leu
              515             520             525

gct ggc tca tat aca aaa cag gac tac ata gat agc atg gaa ggg gca
1814

Ala Gly Ser Tyr Thr Lys Gln Asp Tyr Ile Asp Ser Met Glu Gly Ala
              530             535             540

act ctt tca ggt agg caa gca tct gca tac gta tgt gat gct ggc gag
1862

Thr Leu Ser Gly Arg Gln Ala Ser Ala Tyr Val Cys Asp Ala Gly Glu
          545             550             555

aag ctg gtg gtg ttg cgg aaa aag att gct gct gct gag tca aac gag
1910

Lys Leu Val Val Leu Arg Lys Lys Ile Ala Ala Ala Glu Ser Asn Glu
    560             565             570
```

27

```
atc tct gaa ggt gta tca gta tct gat gag ttg agt ctt gtc tga
1955

Ile Ser Glu Gly Val Ser Val Ser Asp Glu Leu Ser Leu Val
575             580             585

tgactggaaa tcatccaatg aatactgaag agcacccccc actttgttaa tccgagaagc
2015

agatacaaac ataactcagt taggcattgc gtaaggaaga gttcttctaa attttgagtt
2075

cacaagatgg aaatcaaaag gttaaaatat gttgtatgta atattagtaa atcttcatag
2135

tgatgtatct attctgccac ccttcaggtt tagtgaaatg gatcgtattg ctcatcattc
2195

attgagaaga
2205
```

```
<210> 6
<211> 588
<212> PRT
<213> Nicotiana tabacum

<400> 6
Met Ala Thr Ser Ser Ala Tyr Leu Cys Cys Pro Ala Thr Ser Ala Thr
  1               5                  10                  15

Gly Lys Lys His Ile Leu Pro Asn Gly Ser Ala Gly Phe Leu Val Phe
             20                  25                  30

Arg Gly Pro Arg Leu Ser Asn Arg Phe Val Thr Arg Lys Ser Val Ile
         35                  40                  45

Arg Ala Asp Leu Asp Ser Met Val Ser Asp Met Ser Thr Asn Ala Pro
     50                  55                  60

Lys Gly Leu Phe Pro Pro Glu Pro Glu His Tyr Arg Gly Pro Lys Leu
 65                  70                  75                  80

Lys Val Ala Ile Ile Gly Ala Gly Leu Ala Gly Met Ser Thr Ala Val
                 85                  90                  95

Glu Leu Leu Asp Gln Gly His Glu Val Asp Ile Tyr Glu Ser Arg Pro
             100                 105                 110

Phe Ile Gly Gly Lys Val Gly Ser Phe Val Asp Arg Arg Gly Asn His
         115                 120                 125

Ile Glu Met Gly Leu His Val Phe Phe Gly Cys Tyr Asn Asn Leu Phe
     130                 135                 140

Arg Leu Leu Lys Lys Val Gly Ala Glu Lys Asn Leu Leu Val Lys Asp
145                 150                 155                 160

His Thr His Thr Phe Val Asn Lys Gly Gly Glu Ile Gly Glu Leu Asp
                 165                 170                 175

Phe Arg Phe Pro Val Gly Ala Pro Leu His Gly Ile Asn Ala Phe Leu
             180                 185                 190
```

```
Ser Thr Asn Gln Leu Lys Ile Tyr Asp Lys Ala Arg Asn Ala Val Ala
        195                 200                 205

Leu Ala Leu Ser Pro Val Val Arg Ala Leu Val Asp Pro Asp Gly Ala
        210                 215                 220

Leu Gln Gln Ile Arg Asp Leu Asp Ser Val Ser Phe Ser Glu Trp Phe
225                 230                 235                 240

Met Ser Lys Gly Gly Thr Arg Ala Ser Ile Gln Arg Met Trp Asp Pro
                245                 250                 255

Val Ala Tyr Ala Leu Gly Phe Ile Asp Cys Asp Asn Ile Ser Ala Arg
                260                 265                 270

Cys Met Leu Thr Ile Phe Ala Leu Phe Ala Thr Lys Thr Glu Ala Ser
        275                 280                 285

Leu Leu Arg Met Leu Lys Gly Ser Pro Asp Val Tyr Leu Ser Gly Pro
        290                 295                 300

Ile Lys Lys Tyr Ile Leu Asp Lys Gly Gly Arg Phe His Met Arg Trp
305                 310                 315                 320

Gly Cys Arg Gln Val Leu Tyr Glu Thr Ser Ser Asp Gly Ser Met Tyr
                325                 330                 335

Val Ser Gly Leu Ala Met Ser Lys Ala Thr Gln Lys Lys Val Val Lys
        340                 345                 350

Ala Asp Ala Tyr Val Ala Ala Cys Asp Val Pro Gly Ile Lys Arg Leu
        355                 360                 365

Val Pro Gln Lys Trp Arg Glu Leu Glu Phe Phe Asp Asn Ile Tyr Lys
        370                 375                 380

Leu Val Gly Val Pro Val Val Thr Val Gln Leu Arg Tyr Asn Gly Trp
385                 390                 395                 400

Val Thr Glu Leu Gln Asp Leu Glu Arg Ser Arg Gln Leu Lys Arg Ala
                405                 410                 415

Thr Gly Leu Asp Asn Leu Leu Tyr Thr Pro Asp Ala Asp Phe Ser Cys
        420                 425                 430

Phe Ala Asp Leu Ala Leu Ala Ser Pro Glu Asp Tyr Tyr Ile Glu Gly
        435                 440                 445

Gln Gly Ser Leu Leu Gln Cys Val Leu Thr Pro Gly Asp Pro Tyr Met
        450                 455                 460

Pro Leu Leu Asn Asp Glu Ile Ile Lys Arg Val Ser Lys Gln Val Leu
465                 470                 475                 480

Ala Leu Phe Pro Ser Ser Gln Gly Leu Glu Val Thr Trp Ser Ser Val
                485                 490                 495

Val Lys Ile Gly Gln Ser Leu Tyr Arg Glu Gly Pro Gly Lys Asp Pro
                500                 505                 510

Phe Arg Pro Asp Gln Lys Thr Pro Val Glu Asn Phe Phe Leu Ala Gly
        515                 520                 525
```

29

```
Ser Tyr Thr Lys Gln Asp Tyr Ile Asp Ser Met Glu Gly Ala Thr Leu
    530                 535                 540

Ser Gly Arg Gln Ala Ser Ala Tyr Val Cys Asp Ala Gly Glu Lys Leu
545                 550                 555                 560

Val Val Leu Arg Lys Lys Ile Ala Ala Ala Glu Ser Asn Glu Ile Ser
                565                 570                 575

Glu Gly Val Ser Val Ser Asp Glu Leu Ser Leu Val
                580                 585
```

```
<210> 7
<211> 24
<212> DNA
<213> Nicotiana tabacum

<400> 7
aacccgggat agcacgattc aatg                                24


<210> 8
<211> 25
<212> DNA
<213> Nicotiana tabacum

<400> 8
aacccgggat ttccagtcat cagac                               25


<210> 9
<211> 22
<212> DNA
<213> Nicotiana tabacum

<400> 9
ttcccgggct cagtaaaatg cc                                  22


<210> 10
<211> 23
<212> DNA
<213> Nicotiana tabacum

<400> 10
tacccgggct aaactacgct tgc                                 23
```

**Beschreibung**

Die Erfindung betrifft Nukleinsäuren, die für die die Zeta Carotene Synthase aus Tabak kodieren, davon kodierte Polypeptide sowie Verfahren zum Auffinden von Modulatoren der Aktivität der Zeta Carotene Synthase, der Phytoene Synthase und der Phytoene Desaturase.

Unerwünschtes Pflanzenwachstum kann durch die Verwendung von Herbiziden verhindert werden. Die Ansprüche an Herbizide sind dabei hinsichtlich ihrer Wirksamkeit, Kosten und Umweltverträglichkeit stetig angestiegen. Es existiert deshalb ein Bedarf an neuen Substanzen, die zu leistungsfähigen neuen Herbiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt.

Vorteilhafte Angriffspunkte für Herbizide werden in pflanzenspezifischen Biosynthesewegen gesucht, die in tierischen Organismen nicht vorkommen. Ein Beispiel dafür ist der Carotenoid-Biosyntheseweg.

Die Carotinoide haben im Stoffwechsel der Pflanzen vielfältige Bedeutung. Sie sind im photosynthetischen Apparat mit dem *Light Harvesting Complex* assoziiert, der die optimale Weiterleitung der einstrahlenden Photonen an die photosynthetischen Reaktionszentren garantiert. Des weiteren sind sie an der Dissipation überschüssiger Lichtenergie und dem Abfangen von Sauerstoffradikalen beteiligt, und besitzen demnach eine Schutzfunktion. Neben der Bedeutung für die Photosynthese sind die Carotinoide Precursor für die Biosynthese der Xantophylle und den Wachstumsregulator Abscisinsäure. In den Blüten und Früchten fungieren die Carotinoide als Farbpigmente, beispielsweise das Lycopin in der Tomate *(Lycopersicon esculentum)* oder das β-Carotin in der Karotte (*Daucus carota*).

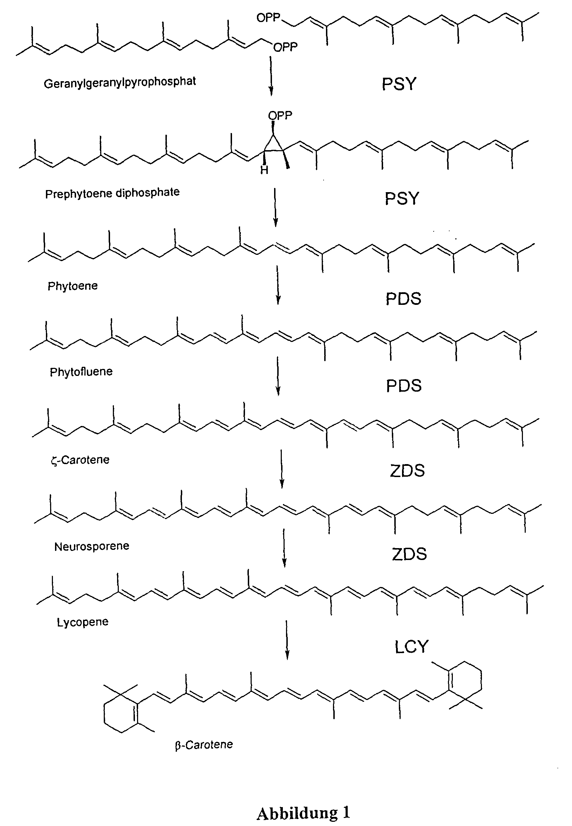

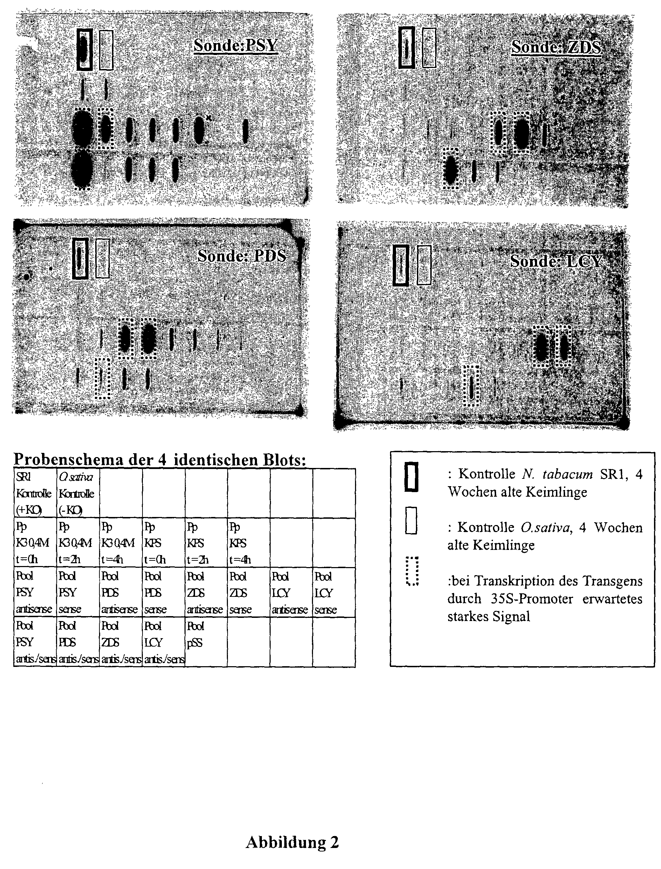

Die Biosynthese der Carotinoide findet in den Plastiden statt. Der Precursor für die Synthese ist das Diterpen Geranylgeranylpyrophosphat. Es wird vermutlich über den sogenannten DXP- oder auch Rohmer-Pathway gebildet und nicht über den klassischen Mevalonat-Weg (Lichtenthaler, 1997). Zwei Moleküle Geranylgeranylpyrophosphat (GGPP) werden durch die Phytoene Synthase (PSY) in einer Kopf-Kopf-Kupplung zu einem Molekül Phytoen umgesetzt (Abbildung 1). Durch die Enzyme Phytoene Desaturase (PDS) und Zeta Carotene Desaturase (ZDS) werden sukzessive vier weitere Doppelbindungen eingeführt. Dabei katalysiert jedes Enzym die Dehydrierung an symmetrischen Positionen (Abbildung 1).

Das entstehende Lycopin wird dann in einem ersten Schritt entweder durch die Lycopene ε-Cyclase (LCYe) zu δ-Carotin oder die Lycopene β-Cyclase (LCYb) zu γ-Carotin umgesetzt. In einem zweiten Schritt entstehen daraus α- respektive β-Carotin. Diese zweite Zyklisierung wird jeweils durch die Lycopene β-Cyclase durchgefürt. α- und β-Carotin bilden dann die Precursor für die Synthese der Xantophylle wie Lutein, Astaxanthin, Violaxanthin u.s.w..

Sämtliche an der Biosynthese der Carotinoide und Xanthophylle beteiligte Enzyme sind nukleär kodiert. Von fast allen Proteinen sind die Gensequenzen aus mindestens einer Pflanze bekannt. Das translatierte Präprotein wird über eine Erkennungssequenz, das Transitpeptid, in den Chloroplasten importiert, prozessiert, oligomerisiert und gegebenenfalls transloziert. Dieser Prozess findet unter Beteiligung des Chaperons Cpn60 und des Heat-Shock-Proteins Hsp70 statt (Bonk, 1997).

Die Phytoene Synthase ist ein Protein von ~45 kDa. Die PSY benötigt als Cofaktoren Manganionen und ATP (Dogbo, 1988). Des weiteren ist für die Aktivität des pflanzlichen Enzyms die Assoziation an Galactolipide erforderlich. Bei der Überexpression der PSY aus *Erwinia uredovora* wurde eine Sensitivität gegenüber Phosphat und eine Inhibierbarkeit durch Squalestatin mit einem pI-Wert von 15 μM gemessen (Neudert, 1998). Das korreliert mit der Homologie auf Aminosäureebene von ~34 % der bekannten PSY-Gene zu Squalensynthasen und weist auf ein enge Verwandtschaft dieser beiden Enzyme hin. Die Synthese von Squalen läuft über die analoge Kopf-Kopf-Kupplung von zwei Molekülen Farnesylpyrophosphat ab, die mechanistisch der Phytoensynthese entspricht. Für die Phytoene Synthase aus *Capsicum anuum* konnte eine lichtabhängige Induktion der Expression nachgewiesen werden (Lintig, 1997). Die Überexpression der fruchtspezifischen PSY1 in Tomatenpflanzen führte phänotypisch zu Zwergenwuchs. Dies wurde auf die Umleitung von Geranylgeranylpyrophosphat aus der Gibberellin- in die Carotinoidbiosynthese zurückgeführt (Fray, 1995). DNA kodierend für die PSY, z. B. aus der Melone und aus Nicotiana-Spezies wurde bereits beschrieben (WO 96/02650, US 5,705,624). Bislang ist jedoch über die Bedeutung der Phytoene Synthase für die Vitalität einer Pflanze jedoch nichts bekannt geworden. Ob das Ausschalten des Gens kodierend für die PSY für eine Pflanze letal ist, das Enzym also als Zielmoleküle für herbizid wirksame Substanzen geeignet ist, wurde bislang nicht bekannt.

Die Phytoene Desaturase ist ein Protein von ~64 kDa. Die PDS wird durch Flavinylierung aktiviert und verwendet Plastoquinon als Elektronenakzeptor (Norris, 1995). Über die Regulation der Phytoene Desaturase liegen widersprüchliche Angaben vor. So wird einerseits von einer Beeinflussung der PDS-Genexpression durch den Chlorophyll- und Pigmentgehalt berichtet (Corona, 1996), andererseits die Abhängigkeit der PDS-Expression vom Pigmentgehalt negiert (Woetzel, 1998). Nach Inhibition der PDS mit den bekannten Inhibitoren Norflurazon bzw. Fluridon konnte *in vitro* ein Verlust der Aktivität des Photosystems II nachgewiesen werden. Dieser Aktivitätsverlust wurde auf die Notwendigkeit des Vorhandenseins von β-Carotene für den Einbau des D1-Proteins in ein funktionelles Photosystems II zurückgeführt (Trebst, 1997). Ob das Ausschalten der Phytoene Desaturase für die Pflanze jedoch letal ist, das Enzym also als Zielmolekül für herbizide Wirkstoffe geeignet ist, wurde bislang jedoch nicht untersucht. Die Sequenz der Phytoene Desaturase aus *Nicotiana tabacum* ist im Dokument US 5,539,093 beschrieben. Diese Sequenz soll ausdrücklich Teil der vorliegenden Anmeldung sein. DNA-Sequenzen kodierend für die PDS finden sich unter anderem in der WO 99/55888.

Die Zeta Carotene Desaturase hat eine Größe von ~65 kDa und ist das am wenigsten charakterisierte Enzym der Carotinoid-Biosynthese. Die Sequenzen der bekannten pflanzlichen ZDS, z. B. aus Reis, Mais, Weizen, Soja oder *Capsicum anuum* (WO 99/55888), weisen Homologien um 34 % zu den bekannten PDS-Sequenzen auf. Über die Regulation der ZDS liegen bis dato keine Angaben vor.

Die Lycopene β-Cyclase ist ein Protein mit einer Größe von ~55 kDa. In den Plastiden kompetiert sie mit der Lycopene ε-Cyclase um das gemeinsame Substrat Lycopin. Im Gegensatz zu den β-Cyclasen, von denen die Gene aus verschiedenen Pflanzen bekannt sind, sind bei der ε-Cyclase nur die pflanzlichen Gene aus *Arabidoposis thaliana* und Tomate bekannt. Der Vergleich der Sequenzen der zwei Cyclase-Typen zeigt eine Homologie auf Aminosäureebene von ~36%. Die Zyklisierung des Lycopins durch die beiden Cyclasen stellt einen Verzweigungspunkt der Carotinoidbiosynthese und damit einen sinnvollen Regulationspunkt dar. Unter Starklicht steigt das Verhältnis von β- zu ε-Cyclase und es werden mehr der protektiven Xantophylle Zeaxanthin, Violaxanthin und Antheraxanthin gebildet. Bei Schwachlicht sinkt das Verhältnis von β- zu ε-Cyclase und es wird mehr an der Lichternte beteiligtes Lutein gebildet (Cunningham, 1996).

Die Biosynthese der Cartoinoide wie auch der Xanthophylle stellt einen hochgradig regulierten Prozess dar. In den Chloroplasten der photosynthetischen Gewebe findet eine Regulation der Biosynthese in Abhängigkeit von der Lichtintensität statt. Demgegenüber steht in den Chromoplasten der Blüten und Früchte eine Regulation in Abhängigkeit vom Entwicklungsstadium. In den Früchten der Tomate (*Lycopersicon esculentum*) wird die Rotfärbung im Verlauf der Reifung durch die Akkumulation von Lycopin erreicht. Diese Akkumulation geht einher mit einer Erhöhung der Transkriptmengen der PSY und der PDS. Gleichzeitig verschwinden die Transkripte für die Lycopin-Cyclasen (Pecker, 1996).

Über die genauen Mechanismen der Regulation der Carotinoidbiosynthese ist nur wenig bekannt.

Die vorliegende Anmeldung beschreibt die Klonierung von Genen der Carotinoid-Biosynthese. Dabei wurden unter anderem zwei Phytoene Synthase-Gene gefunden. Eine Analogie zur entwicklungsabhängigen Regulation wie bei der Tomate konnte nicht gezeigt werden. Es besteht die Möglichkeit einer Regulation in Abhängigkeit von der Lichtintensität. In diesem Fall könnte ein Gen die *house keeping* -Aktivität kodieren, das andere hingegen in Abhängigkeit von der Lichtintensität reguliert sein.

Die vorliegende Anmeldung beschreibt ebenfalls die Klonierung des Gens kodierend für die Zeta-Carotene Desaturase.

Die Anmeldung beschreibt ebenfalls die Klonierung des Gens kodierend für die Phytoene Desaturase aus *Nicotiana tabacum*.

Die vorliegende Anmeldung beschreibt ebenfalls, dass die bekannten Enzyme des Carotinoid-Biosyntheseweges, die Phytoene Synthase, die Phytoene Desaturase sowie die Zeta Carotene Desaturase, die durch die Katalyse aufeinander folgender Schritte bei der Biosynthese von Carotenoiden miteinander in Zusammenhang stehen, von essentieller Bedeutung für Pflanzen sind. Die vorliegende Anmeldung beschreibt ebenfalls, dass die Enzyme Phytoene Synthase, Phytoene Desaturase sowie Zeta Carotene Desaturase als Zielmoleküle (Targets) für herbizide Wirkstoffe geeignet sind und deshalb in Verfahren zum Auffinden von herbiziden Wirkstoffen verwendet werden können.

Gegenstand der vorliegenden Erfindung ist demnach die Verwendung der Enzyme der Carotenoid-Biosynthese, der Phytoene Synthase, der Phytoene Desaturase und der Zeta Carotene Desaturase, in Verfahren zum Auffinden von herbizid wirksamen Substanzen.

Gegenstand der vorliegenden Erfindung sind demnach Nukleinsäuren, die für pflanzliche Polypeptide mit der biologischen Aktivität einer Phytoene Synthase, welche die Aminosäuresequenz gemäß SEQ ID NO: 2 oder SEQ ID NO: 4 umfasst, kodieren. Insbesondere kodieren die erfindungsgemäßen Nukleinsäuren für die Phytoene Synthase aus Tabak, wobei die erfindungsgemäßen Nukleinsäuren aus *Nicotiana tabacum* SR1 besonders bevorzugt sind.

Gegenstand der vorliegenden Erfindung sind ebenfalls Fragmente der erfindungsgemäßen Nukleinsäuren, die für die Phytoene Synthase kodieren.

Gegenstand der vorliegenden Erfindung sind ebenfalls Nukleinsäuren, die für pflanzliche Polypeptide mit der biologischen Aktivität einer Zeta-Carotene Desaturase, welche die Aminosäuresequenz gemäß SEQ ID NO. 6: umfasst, kodieren. Insbesondere kodieren die erfindungsgemäßen Nukleinsäuren für die Zeta-Carotene Desaturase aus Tabak, wobei die erfindungsgemäßen Nukleinsäuren aus *Nicotiana tabacum* SR1 besonders bevorzugt sind.

Gegenstand der vorliegenden Erfindung sind ebenfalls Fragmente der erfindungsgemäßen Nukleinsäuren, die für die Zeta-Carotene Desaturase kodieren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die gegebenenfalls auch Introns enthalten können, und cDNAs.

Die Fragmente können dabei ebenfalls einzelsträngig oder doppelsträngig sein, wobei einzelsträngige Fragmente komplementär zum kodogenen oder zum kodierenden Strang der erfindungsgemäßen Nukleinsäuren sein können. Solche einzelsträngigen Fragmente können dann jeweils an den kodogenen oder an den kodierenden Strang der erfindungsgemäßen Nukleinsäure hybridisieren.

Der Ausdruck "Fragment", wie er hierin verwendet wird, umfasst dabei einzelsträngige oder doppelsträngige Nukleinsäuren einer Länge von 10 bis 1 000 Basenpaaren (bp), bevorzugt einer Länge von 12 bis 500 bp, besonders bevorzugt einer Länge von 15 bis 200 bp und ganz besonders bevorzugt einer Länge von 20 bis 100 Basenpaaren.

Bevorzugt handelt es sich bei den erfindungsgemäßen Nukleinsäuren um DNA, die der genomischen DNA von Tabakpflanzen, die Introns enthalten kann, oder Fragmenten davon entspricht.

Besonders bevorzugt umfassen die erfindungsgemäßen Nukleinsäuren eine Sequenz ausgewählt aus

a) der Sequenz gemäß SEQ ID NO: 1, 3 oder 5

b) Sequenzen, die für ein Polypeptid kodieren, welches die Aminosäuresequenz gemäß SEQ ID NO: 2, 4 oder 6 umfasst,

c) zumindest 14 Basenpaare langen Teilsequenzen der unter a) oder b) definierten Sequenzen,

d) Sequenzen, welche an die unter a), b) oder c) definierten Sequenzen hybridisieren,

e) Sequenzen, welche zu den unter a), b) oder c) definierten Sequenzen komplementär sind, und

f) Sequenzen, welche aufgrund der Degeneriertheit des genetischen Codes für dieselbe Aminosäuresequenz kodieren wie die unter a) bis c) definierten Sequenzen.

Eine ganz besonders bevorzugte Ausführungsform der erfindungsgemäßen Nukleinsäuren stellt ein cDNA-Molekül mit der Sequenz gemäß SEQ ID NO: 1 dar.

Eine weitere ganz besonders bevorzugte Ausführungsform der erfindungsgemäßen Nukleinsäuren stellt ein cDNA-Molekül mit der Sequenz gemäß SEQ ID NO: 3 dar.

Eine weitere banz besonders bevorzugte Ausführungsform der erfindungsgemäßen Nukleinsäuren stellt ein cDNA-Molekül mit der Sequenz gemäß SEQ ID NO: 5 dar.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin offenbarten Sequenzinformation beispielsweise DNA-Fragmente aus anderen Pflanzen als Tabakpflanzen isoliert werden, welche für die Phytoene Synthase oder die Zeta Carotene Desaturase kodieren, welche dieselben oder ähnliche Eigenschaften wie die Enzyme mit der Aminosäuresequenz gemäß SEQ ID NO: 2 oder 4, bzw. SEQ ID NO: 5 aufweisen.

Hybridisierungsbedingungen werden nach folgender Formel näherungsweise berechnet:

Die Schmelztemperatur

$$Tm = 81{,}5°C + 16{,}6 \log\{c(Na^+)] + 0{,}41(\%G + C)) - 500/n$$

(Lottspeich und Zorbas, 1998).

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM $Na^+$ (entspricht 0.1 x SSC) gewaschen. Wird eine RNA-Probe zur Hybridisierung verwendet, so ist der Schmelzpunkt um 10 bis 15°C höher.

Bevorzugte Hybridisierungsbedingungen sind nachstehend angegeben:

Hybridisierungslösung: 6x SSC / 5x Denhardt's Lösung / 50 % Formamid;
Hybridisierungstemperatur: 36°C, bevorzugt 42°C;

1. Waschschritt: 2x SSC, 30 min bei Raumtemperatur;
2. Waschschritt: 1x SSC, 30 min bei 50°C; bevorzugt 0,5x SSC, 30 min bei 65°C; besonders bevorzugt 0,2x SSC, 30 min bei 65°C.

Der Grad der Identität der Nukleinsäuren wird vorzugsweise bestimmt mit Hilfe des Programms NCBI BLASTN Version 2.0.4. (Altschul et al., 1997).

Gegenstand der vorliegenden Erfindung sind auch die regulatorischen Regionen, welche natürlicherweise in Pflanzenzellen, insbesondere in Tabakpflanzen, die Transkription der erfindungsgemäßen Nukleinsäuren kontrollieren.

Der Ausdruck "regulatorische Regionen", wie er hierin verwendet wird, bezieht sich auf nicht-translatierte Regionen des betreffenden Gens, wie Promotoren, Enhancer, Repressor- oder Aktivator-Bindungsstellen oder Terminationssequenzen, die mit zellulären Proteinen interagieren, wodurch die Transkription gesteuert wird.

Gegenstand der vorliegenden Erfindung sind weiterhin DNA-Konstrukte, die eine erfindungsgemäße Nukleinsäure und einen heterologen Promotor umfassen.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promoter des Blumenkohlmosaikvirus für pflanzliche Zellen, der Promoter der Alkoholdehydrogenase für Hefezellen, die T3-, T7- oder SP6-Promotoren für prokaryotische Zellen oder zellfreie Systeme.

Gegenstand der vorliegenden Erfindung sind ferner Vektoren, die eine erfindungsgemäße Nukleinsäure, eine erfindungsgemäße regulatorische Region oder ein erfindungsgemäßes DNA-Konstrukt enthalten. Als Vektoren können alle in molekularbiologischen Laboratorien verwendeten Phagen, Plasmide, Phagmide, Phasmide, Cosmide, YACs, BACs, künstliche Chromosomen oder Partikel, die für einen Partikelbeschuss geeignet sind, verwendet werden.

Bevorzugte Vektoren sind pBIN (Bevan, 1984) und seine Derivate für pflanzliche Zellen, pFL61 (Minet et al., 1992) für Hefezellen, pBLUESCRIPT-Vektoren für bakterielle Zellen, lamdaZAP (Fa. Stratagene) für Phagen.

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, vorzugsweise *E. coli*, als auch eukaryotische Zellen, wie Zellen von *Saccharomyces cerevisiae*, *Pichia pastoris*, Insekten, Pflanzen, Froschoozyten und Zelllinien von Säugern.

Gegenstand der vorliegenden Erfindung sind weiterhin Polypeptide mit der biologischen Aktivität einer Phytoene Synthase, die von den erfindungsgemäßen Nukleinsäuren kodiert werden. Insbesondere handelt es sich um Polypeptide, die erfindungsgemäße Phytoene Synthasen darstellen. Ganz besonders sind die Polypeptide Gegenstand dieser Erfindung, die einer Aminosäuresequenz gemäß SEQ ID NO: 2 oder SEQ ID NO: 4 entsprechen.

Gegenstand der vorliegenden Erfindung sind weiterhin Polypeptide mit der biologischen Aktivität einer Zeta Carotene Desaturase, die von den erfindungsgemäßen Nukleinsäuren kodiert werden. Insbesondere handelt es sich um Polypeptide, die erfindungsgemäße Zeta Carotene Desaturasen darstellen. Ganz besonders sind die Polypeptide Gegenstand dieser Erfindung, die einer Aminosäuresequenz gemäß SEQ ID NO: 6 entsprechen.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Amino-und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen.

Die erfindungsgemäßen Polypeptide müssen nicht eine vollständige Phytoene Synthase bzw. Zeta Carotene Desaturase darstellen, sondern können auch nur Fragmente davon sein, solange sie zumindest noch eine biologische Aktivität der vollständigen Phytoene Synthase bzw. Zeta Carotene Desaturase aufweisen. Solche Fragmente, die eine gleichartige biologische Aktivität wie eine Phytoene Synthase mit einer Aminosäuresequenz gemäß SEQ ID NO: 2 oder SEQ ID NO: 4 oder eine gleichartige biologische Aktivität wie eine Zeta Carotene Desaturase mit einer Aminosäuresequenz gemäß SEQ ID NO: 6 ausüben, werden als erfindungsgemäß betrachtet.

Die erfindungsgemäßen Polypeptide können im Vergleich zu den entsprechenden Regionen von natürlich vorkommenden Phytoene Synthasen bzw. Zeta Carotene Desaturasen aus Tabak Deletionen oder Aminosäuresubstitutionen aufweisen, solange sie zumindest noch eine biologische Aktivität der vollständigen Enzyme ausüben. Konservative Substitutionen sind bevorzugt. Solche konservativen Substitutionen umfassen Variationen, wobei eine Aminosäure durch eine andere Aminosäure aus der folgenden Gruppe ersetzt wird:

1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| Ursprünglicher Rest | Substitution |
| --- | --- |
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Eine bevorzugte Ausführungsform der erfindungsgemäßen Polypeptide ist die Phytoene Synthase aus Tabak mit der Aminosäuresequenz gemäß SEQ ID NO: 2 oder der Aminosäuresequenz gemäß SEQ ID NO: 4.

Eine weitere bevorzugte Ausführungsform der erfindungsgemäßen Polypeptide ist die Zeta Carotene Desaturase aus Tabak mit der Aminosäuresequenz gemäß SEQ ID NO: 6.

Gegenstand der vorliegenden Erfindung sind weiterhin Antikörper, die spezifisch an die erfindungsgemäßen Polypeptide binden. Die Herstellung solcher Antikörper erfolgt auf die übliche Weise. Diese Antikörper können beispielsweise dazu genutzt werden, um Expressionsklone, z.B. einer Genbank, zu identifizieren, die die erfindungsgemäßen Nukleinsäuren tragen.

Der Ausdruck "Antikörper", wie er hierin verwendet wird, erstreckt sich auch auf Teile vollständiger Antikörper, wie Fa-, F(ab')$_2$- oder Fv-Fragmente, welche noch die Fähigkeit besitzen, an die Epitope der erfindungsgemäßen Polypeptide zu binden.

Weiterhin sind auch Verfahren zum Herstellen der erfindungsgemäßen Nukleinsäuren Gegenstand der vorliegenden Erfindung. Die erfindungsgemäßen Nukleinsäuren können auf die übliche Weise hergestellt werden. Beispielsweise können die Nukleinsäuremoleküle vollständig chemisch synthetisiert werden. Man kann auch kurze Stücke der erfindungsgemäßen Nukleinsäuren chemisch synthetisieren und solche Oligonukleotide radioaktiv oder mit einem Fluoreszenzfarbstoff markieren. Die markierten Oligonukleotide können auch verwendet werden, um ausgehend von Pflanzen-mRNA hergestellte cDNA-Banken zu durchsuchen. Klone, an die die markierten Oliogonukleotide hybridisieren, werden zur Isolierung der betreffenden DNA-Fragmente ausgewählt. Nach der Charakterisierung der isolierten DNA erhält man auf einfache Weise die erfindungsgemäßen Nukleinsäuren.

Die erfindungsgemäße Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Gegenstand der vorliegenden Erfindung sind weiterhin Verfahren zum Herstellen der erfindungsgemäßen Polypeptide. Zur Herstellung der Polypeptide, die von den erfindungsgemäßen Nukleinsäuren codiert werden, können Wirtszellen, die erfindungsgemäße Nukleinsäuren enthalten, unter geeigneten Bedingungen kultiviert werden. Die gewünschten Polypeptide können danach auf übliche Weise aus den Zellen oder dem Kulturmedium isoliert werden. Die Polypeptide können auch in *in-vitro*-Systemen hergestellt werden.

Ein schnelles Verfahren zum Isolieren der erfindungsgemäßen Polypeptide, die von Wirtszellen unter Verwendung einer erfindungsgemäßen Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Gegenstand der vorliegenden Erfindung sind auch Verfahren zum Auffinden von chemischen Verbindungen, die an die erfindungsgemäßen Polypeptide binden und deren Eigenschaften verändern. Solche Verbindungen können als Modulatoren der erfindungsgemäßen Polypeptide wirken, entweder als Agonisten oder als Antagonisten.

Gegenstand der vorliegenden Erfindung sind auch Verfahren zum Auffinden von chemischen Verbindungen, die an die Phytoene Desaturase binden und deren Eigenschaften verändern, wobei diese Verbindungen als Agonisten oder Antagonisten wirken können

Der Ausdruck "Agonist", wie er hierin verwendet wird, bezieht sich auf ein Molekül, das die enzymatische Aktivität des Enzyms Phytoene Synthase, des Enzyms Phytoene Desaturase oder des Enzyms Zeta Carotene Desaturase beschleunigt oder verstärkt.

Der Ausdruck "Antagonist", wie er hierin verwendet wird, bezieht sich auf ein Molekül, das die enzymatische Aktivität des Enzyms Phytoene Synthase, des Enzyms Phytoene Desaturase oder des Enzyms Zeta Carotene Desaturase verlangsamt oder inhibiert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon.

Vorzugsweise handelt es sich bei den Modulatoren um kleine organisch-chemische Verbindungen.

Die Bindung der Modulatoren an die Enzyme Phytoene Synthase, Phytoene Desaturase und Zeta-Carotene Desaturase kann die zellulären Vorgänge auf eine Weise verändern, die zum Absterben der damit behandelten Pflanzen führt.

Die vorliegende Erfindung umfasst damit auch die Verwendung der erfindungsgemäßen Polypeptide Phytoene Synthase, Zeta Carotene Desaturase sowie der Phytoene Desaturase in Verfahren zum Auffinden von Verbindungen, die die Aktivität der Enzyme beeinflussen.

Weiterhin umfasst die vorliegende Erfindung Verfahren zum Auffinden von chemischen Verbindungen, welche die Expression der Phytoene Synthase, Zeta Carotene Desaturase sowie der Phytoene Desturase verändern. Auch solche "Expressionsmodulatoren" können neue wuchsregulierende oder herbizide Wirkstoffe darstellen. Expressionsmodulatoren können kleine organisch-chemische Moleküle, Peptide oder Antikörper sein, die an die regulatorischen Regionen der für die Phytoene Synthase, Zeta Carotene Desaturase oder die Phytoene Desturase kodierenden Nukleinsäuren binden. Weiterhin können Expressionsmodulatoren kleine organisch-chemische Moleküle, Peptide oder Antikörper sein, die an ein Molekül binden, welches wiederum an regulatorische Regionen der für die Phytoene Synthase, Zeta Carotene Desaturase sowie der Phytoene Desaturase kodierenden Nukleinsäuren bindet, und dadurch deren Expression beeinflusst. Expressionsmodulatoren können auch Antisense-Moleküle sein.

Daher erstreckt sich die vorliegende Erfindung auch auf die Verwendung von Modulatoren der Phytoene Synthase, Zeta Carotene Desaturase und der Phytoene Desaturase oder von Expressionsmodulatoren als Pflanzenwuchsregulatoren oder Herbizide.

Die erfindungsgemäßen Verfahren schließen Hochdurchsatz-Screening (high throughput screening; HTS) ein. Dafür können sowohl Wirtszellen als auch zellfreie Präparationen verwendet werden, die die erfindungsgemäßen Nukleinsäuren und/oder die Phytoene Synthase, Zeta Carotene Desaturase oder die Phytoene Desaturase oder dafür kodierende Nukleinsäuren enthalten.

Um Modulatoren aufzufinden, kann ein synthetischer Reaktionsmix (z.B. Produkte der *in vitro*-Transkription) oder ein zellulärer Bestandteil, der die Phytoene Synthase, Zeta Carotene Desaturase oder die Phytoene Desaturase enthält, zusammen mit einem markierten Substrat oder Liganden der Polypeptide in Gegenwart und Abwesenheit eines Kandidatenmoleküls, das ein Agonist oder Antagonist sein kann, inkubiert werden. Die Fähigkeit des Kandidatenmoleküls die Aktivität der Phytoene Synthase, Zeta Carotene Desaturase oder der Phytoene Desaturase zu erhöhen oder zu hemmen, wird erkennbar an einer erhöhten oder verringerten Bindung des markierten Liganden oder an einer erhöhten oder verringerten Umsetzung des markierten Substrates. Moleküle, die gut binden und zu einer erhöhten Aktivität der Phytoene Synthase, Zeta Carotene Desaturase oder der Phytoene Desaturase führen, sind Agonisten. Moleküle, die gut binden, aber nicht die biologische Aktivität der Phytoene Synthase, Zeta Carotene Desaturase oder der Phytoene Desaturase auslösen, sind wahrscheinlich gute Antagonisten. Die Detektion der biologischen Aktivität der Phytoene Synthase, Zeta Carotene Desaturase oder der Phytoene Desaturase kann durch ein sogenanntes Reportersystem verbessert werden. Reportersysteme in dieser Hinsicht umfassen, sind aber nicht beschränkt auf colorimetrisch markierte Substrate, die in ein Produkt umgewandelt werden oder ein Reportergen, das auf Veränderungen der Aktivität oder der Expression der Phytoene Synthase, Zeta Carotene Desaturase oder der Phytoene Desaturase anspricht oder andere bekannte Bindungstests.

Ein weiteres Beispiel für ein Verfahren, mit welchem Modulatoren der erfindungsgemäßen Polypeptide und/oder der Phytoene Desaturase aufgefunden werden können, ist ein Verdrängungstest, bei dem man unter dafür geeigneten Bedingungen die Phytoene Synthase, Zeta Carotene Desaturase oder die Phytoene Desaturase und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide oder die Phytoene Desaturase selbst können markiert werden, z.B. radioaktiv oder colorimetrisch, so dass man die Anzahl der Polypeptide, die an einen Liganden gebunden sind oder die eine Umsetzung mitgemacht haben, exakt bestimmen kann. Auf diese Weise lässt sich die Effektivität eines Agonisten oder Antagonisten ermessen.

Gegenstand der Erfindung ist weiterhin die Verwendung einer erfindungsgemäßen Nukleinsäure, eines erfindungsgemäßen DNA-Konstrukts oder eines erfindungsgemäßen Vektors zum Herstellen von transgenen Pflanzen, sowie die entsprechenden transgenen Pflanzen als solche bzw. deren Teile oder Vermehrungsmaterial.

Transgene Pflanzen, Pflanzenteile, Protoplasten, Pflanzengewebe oder Pflanzenvermehrungsmaterialien, in denen nach Einbringen einer erfindungsgemäßen Nukleinsäure, eines erfindungsgemäßen DNA-Konstrukts oder eines erfindungsgemäßen Vektors die intrazelluläre Konzentration der rezeptorähnlichen Proteinkinasen im Vergleich zu den entsprechenden Wildtypformen erhöht oder vermindert ist, sind ebenfalls Gegenstand der vorliegenden Erfindung.

Der Ausdruck "Pflanzenteile", wie er hierin verwendet wird, bedeutet alle oberirdischen und unterirdischen Teile und Organe der Pflanzen, wie Spross, Blatt, Blüte und Wurzel, sowie daraus hergestellte Protoplasten und Gewebekulturen.

Der Ausdruck "Vermehrungsmaterial", wie er hierin verwendet wird, bedeutet vegetatives und generatives Vermehrungsmaterial, wie Stecklinge, Knollen, Rhizome, Ableger und Samen.

Gegenstand der Erfindung sind auch Pflanzen, in denen Veränderungen an der die Phytoene Synthase kodierenden Sequenz vorgenommen werden und die Pflanzen, die zur Herstellung einer erfindungsgemäßen Phytoene Synthase führen, selektiert werden, oder in denen durch Mutagenese eine Erhöhung oder. Verminderung der endogenen Phytoene Synthaseaktivität erreicht wird.

Gegenstand der Erfindung sind ebenfalls Pflanzen, in denen Veränderungen an der die Zeta-Carotene Desaturase kodierenden Sequenz vorgenommen werden und die Pflanzen, die zur Herstellung einer erfindungsgemäßen Zeta-Carotene Desaturase führen, selektiert werden, oder in denen durch Mutagenese eine Erhöhung oder Verminderung der endogenen Zeta-Carotene Desaturaseaktivität erreicht wird.

## Beispiele

## Beispiel 1

Klonierung von Phytoene Synthase-Genen aus Tabak

Die Klonierung der 5'- und 3'-Termini der PSY erfolgte über RACE-PCR. Die erhaltenen Fragmente wurden sequenziert. Aus den Sequenzen der Fragmente wurde die vollständige Sequenz für die PSY zusammengesetzt. Von den sequenzierten Fragmenten zeigte jeweils ein mittleres und ein 3'-terminales Fragment eine abweichende Sequenz mit einer Homologie von ungefähr 80 % auf Nukleotidebene gegenüber den sonstigen Fragmenten. Im überlappenden Bereich waren diese beiden Fragmente 100 % identisch. Die Tatsache der Übereinstimmung der zwei abweichenden Fragmente aus verschiedenen PCR-Reaktionen implizierte das Vorhandensein eines weiteren PSY-Gens in Tabak. Die bereits vollständige PSY-Sequenz bekam daher den Zusatz PSY1, die abweichende Sequenz den Zusatz PSY2. Entsprechend der partiellen Sequenz der PSY2 wurde ein neuer Primer für die RACE-Amplifikation des entsprechenden 5'-Terminus definiert. Unter Verwendung dieses Primers wurden 5'-terminale Fragmente erhalten. Die Sequenzen dieser Fragmente wurden bestimmt. Sie zeigten 100% Homologie im überlappenden Berich zu der bereits vorliegenden Sequenz der PSY2. Aus den Sequenzen der Fragmente wurde die vollständige Sequenz der PSY2 zusammengesetzt. Auf Basis der erhaltenen Sequenzen wurden die Proteinsequenzen ermittelt. Das gefundene offene Leseraster für die PSY1 kodiert ein Protein von 439 Aminosäuren entsprechend ∼48 kDa. Das offene Leseraster für die PSY2 kodiert ein Protein von 410 Aminosäuren entsprechend ∼45 kDa. Die beiden PSY-Gene aus Tabak zeigen auf Aminosäureebene eine Homologie von 86 %. Dabei liegen die größten Abweichungen in zwei Deletionen von 4 und 22 Aminosäuren im N-terminalen Bereich. Die PSY1 und PSY2 zeigen auf Aminosäureebene Homologien von 96 % respektive 93 % zur PSY2 aus Tomate und von 85 % respektive 87 % zur PSY1 aus Tomate. Aus allen anderen Pflanzen ist jeweils höchstens ein PSY-Gen bekannt.

Die genaue Durchführung der Klonierung wird im Folgenden beschrieben.

Im Gewächshaus wurde Tabaksamen ausgelegt und nach 4 Wochen aus den Keimlingen gesamt-RNA präpariert. Die gesamt-RNA wurde als Vorlage für die Synthese doppelsträngiger cDNA eingesetzt. Die cDNA wurde mit Klenowfragment aufgefüllt und mit T4-Polynukleotidkinase phosphoryliert. An die cDNA wurden Marathon-Adaptoren (5'-ctaatacgac tcactatagg gctcgagcgg ccgcccgggc aggt-3' / 3'-cccg tcca-5') ligiert (Clontech, Advantage cDNA PCR Kit).

Ein Fragment der kodierenden Sequenz der Phytoene Synthase aus *Nicotiana tabacum* wurde mit Hilfe der PCR-Technik mit den Primern der Sequenzen 5'-tatgctaaga cgttttatct tggaac-3' und 5'-ccatacaggc catctgctag c-3' amplifiziert. Das amplifizierte Fragment wurde über TOPO TA-Cloning in den bakteriellen Vektor pCR2.1 (Invitrogen) kloniert. Die Sequenz des amplifizierten Fragments wurde durch Sequenzierung nach Sanger bestimmt. Zwei unterschiedliche Sequenzen wurden erhalten und mit PSY1 und PSY2 benannt.

Der 5'-Terminus der Sequenz der Phytoene Synthase 1 aus *Nicotiana tabacum* wurde mit Hilfe der PCR-Technik mit den Primern der Sequenzen 5'-ccatcgacta gctcatccgt tctcctgcac c-3' und 5'-ccatcctaat acgactcacta tagggc-3' amplifiziert.

Der 5'-Terminus der Sequenz der Phytoene Synthase 2 aus *Nicotiana tabacum* wurde mit Hilfe der PCR-Technik mit den Primern der Sequenzen 5'-aagccggtct tcccacctat ctaaggcttg g-3' und 5'-ccatcctaat acgactcacta tagggc-3' amplifiziert.

Die 3'-Termini der Sequenz der Phytoene Synthase 1 und 2 aus *Nicotiana tabacum* wurden mit Hilfe der PCR-Technik mit den Primern der Sequenzen 5'-agtaggactg atgagtgttc cagttatggg tattgcacc-3' und 5'-ccatcctaat acgactcacta tagggc-3' amplifiziert.

Die amplifizierten Fragmente wurden über TOPO TA-Cloning in den bakteriellen Vektor pCR2.1 (Invitrogen) kloniert. Die Sequenzen der amplifizierten Fragmente wurden durch Sequenzierung nach Sanger bestimmt.

Die transkribierte Sequenz der Phytoene Synthase 1 aus *Nicotiana tabacum* wurde mit Hilfe der PCR-Technik mit den Primern der Sequenzen 5'-agaaacccag aaagaacaac aggttttg-3' und 5'-ctcacttgag ggtttgatga gtgtgg-3' amplifiziert. Das amplifizierte Fragment wurde über TOPO TA-Cloning in den bakteriellen Vektor pCR2.1 (Invitrogen) kloniert. Die Sequenz des amplifizierten Fragments wurde durch Sequenzierung nach Sanger bestimmt. Die Sequenz wurde mit PSY1 benannt.

Die kodierende Sequenz der Phytoene Synthase 1 aus *Nicotiana tabacum* wurde mit Hilfe der PCR-Technik mit den Primern der Sequenzen 5'-ttcccggggt gtttcatgag catg-3' und 5'-ttcccgggtc attcatgtct ttgc-3' amplifiziert. Das amplifizierte Fragment wurde mit der Restriktionsendonuklease *Xma I* nachgeschnitten. Das entstandene *XmaI-*

PSY1-Fragment wurde in den linearisierten und dephosphorylierten Vektor pSS ligiert. Die entstandenen Konstrukte pSS-PSY1 wurden durch Restriktionskartierung auf die Orientierung des Transgens überprüft.

Die kodierende Sequenz der Phytoene Synthase 2 aus *Nicotiana tabacum* wurde mit Hilfe der PCR-Technik mit den Primern der Sequenzen 5'-atgaattctg ttcaaaatgt ctgttgcc-3' und 5'-atgaattcct gatgtctatg ccttagctag ag-3' amplifiziert. Das amplifizierte Fragment wurde mit der Restriktionsendonuklease *Eco*RI nachgeschnitten. Das entstandene *Eco*RI-PSY2-Fragment wurde in den linearisierten und dephosphorylierten Vektor pSS ligiert. Die entstandenen Konstrukte pSS-PSY2 wurden durch Restriktionskartierung auf die Orientierung des Transgens überprüft.

**Beispiel 2**

Klonierung der Zeta-Carotene Desaturase aus *Nicotiana tabacum*

Im Gewächshaus wurde Tabaksamen ausgelegt und nach 4 Wochen aus den Keimlingen gesamt-RNA präpariert. Die gesamt-RNA wurde als Vorlage für die Synthese doppelsträngiger cDNA eingesetzt. Die cDNA wurde mit Klenowfragment aufgefüllt und mit T4-Polynukleotidkinase phosphoryliert. An die cDNA wurden Marathon-Adaptoren (5'-ctaatacgac tcactatagg gctcgagcgg ccgcccgggc aggt-3' / 3'-cccg tcca-5') ligiert (Clontech, Advantage cDNA PCR Kit).

Ein Fragment der kodierenden Sequenz der Zeta-Carotene Desaturase aus *Nicotiana tabacum* wurde mit Hilfe der PCR-Technik mit den Primern der Sequenzen 5'-gagctggact tgcaggcatg tcg-3' und 5'-aactggaaga attcgcggcc gcaggaattt tttttttttt ttttt-3' amplifiziert. Das amplifizierte Fragment wurde über TOPO TA-Cloning in den bakteriellen Vektor pCR2.1 (Invitrogen) kloniert. Die Sequenz des amplifizierten Fragments wurde durch Sequenzierung nach Sanger bestimmt.

Der 5'-Terminus der Sequenz der Zeta-Carotene Desaturase aus *Nicotiana tabacum* wurde mit Hilfe der PCR-Technik mit den Primern der Sequenzen 5'-tccacctcat gtccttgatc caagagctcc-3' und 5'-ccatcctaat acgactcacta tagggc-3' amplifiziert. Das amplifizierte Fragment wurden über TOPO TA-Cloning in den bakteriellen Vektor pCR2.1 (Invitrogen) kloniert. Die Sequenz des amplifizierten Fragments wurde durch Sequenzierung nach Sanger bestimmt.

Die transkribierte Sequenz der Zeta-Carotene Desaturase aus *Nicotiana tabacum* wurde mit Hilfe der PCR-Technik mit den Primern der Sequenzen 5'-ctggcatctt acatctgcca aatttcc-3' und 5'-tcttctcaat gaatgatgag caatacgatc c-3' amplifiziert. Das amplifizierte Fragment wurde über TOPO TA-Cloning in den bakteriellen Vektor pCR2.1 (Invitrogen) kloniert. Die Sequenz des amplifizierten Fragments wurde durch Sequenzierung nach Sanger bestimmt. Die Sequenz SEQ ID NO. 1 wurde erhalten.

Die kodierende Sequenz der Zeta-Carotene Desaturase aus *Nicotiana tabacum* wurde mit Hilfe der PCR-Technik mit den Primern der Sequenzen gemäß SEQ ID NO: 3 und SEQ ID NO: 4 amplifiziert. Das amplifizierte Fragment wurde mit der Restriktionsendonuklease *Xma I* nachgeschnitten. Das entstandene *Xma*I-ZDS-Fragment wurde in den linearisierten und dephosphorylierten Vektor pSS ligiert. Die entstandenen Konstrukte pSS-ZDS wurden durch Restriktionskartierung auf die Orientierung des Transgens überprüft.

Das gefundene offene Leseraster für die ZDS kodiert für ein Protein von 588 Aminosäuren entsprechend ~65 kDa. Die Homologie auf Aminosäureebene zu der bekannten ZDS aus *Capsicum anuum* liegt bei 95%. Es handelt sich demnach um die plastidäre ZDS aus Tabak.

**Beispiel 3**

Klonierung der Phytoene Desaturase aus *Nicotiana tabacum* SR1

Im Gewächshaus wurde Tabaksamen ausgelegt und nach 4 Wochen aus den Keimlingen gesamt-RNA präpariert. Die gesamt RNA wurde als Vorlage für die Synthese einzelsträngiger cDNA eingesetzt (Pharmacia, 1st strand cDNA Synthesis Kit).

Die kodierende Sequenz der Phytoene Desaturase aus *Nicotiana tabacum* wurde mit Hilfe der PCR-Technik mit den Primern der Sequenzen gemäß SEQ ID NO: 9 und SEQ ID NO: 10 amplifiziert. Das amplifizierte Fragment wurde mit der Restriktionsendonuklease *Xma I* nachgeschnitten. Das entstandene *Xma*I-PDS-Fragment wurde in den linearisierten und dephosphorylierten Vektor pSS ligiert. Die entstandenen Konstrukte pSS-PDS wurden durch Restriktionskartierung auf die Orientierung des Transgens überprüft.

**Beispiel 4**

Southern und Northern Blot

Für die weitere Charakterisierung der PSY-Gene aus Tabak wurde ein Southern-Blot durchgeführt. Genomische DNA aus Tabak wurde präpariert und mit verschiedenen Restriktionsendonukleasen geschnitten. Die DNA wurde auf Nitrocellulose geblottet und mit radioaktiv markierter Sonde PSY1 hybridisiert. Es sind jeweils 3 bzw. 4 Banden zu erkennen. Um einen Hinweis auf die Funktionalität der zwei oder gegebenenfalls mehr PSY-Gene in Tabk zu erhalten, wurden Tabak-Samen im Gewächshaus ausgelegt. Nach 2, 4 und 6 Wochen wurde ein Teil des Pflanzenmaterials geemtet und in flüssigem Stickstoff eingefroren. Parallel wurden Blütenblätter von adulten Tabakpflanzen geerntet und ebenso behandelt. Von diesem Material wurden mRNA-Präparationen durchgeführt. Die mRNA wurde auf Nitrocellulose übertragen (Slot Blot) und getrennt mit radioaktiven PSY1- und PSY2-Sonden hybridisiert. Die beiden Gene zeigen insgesamt ein stark unterschiedliches Transkriptions-Niveau. Das Verhältnis der beiden Gene bleibt jedoch unabhängig vom Entwicklungsstadium konstant.

**Beispiel 5**

Ausschalten der für Enzyme des Carotinoid-Biosynthesewegs kodierenden Gene in Tabak

Mit den erhaltenen Sequenzen der PSY, PDS und ZDS sowie der bekannten Sequenz der LCY aus Tabak wurden Primer für die Amplifikation der vollständigen kodierenden Sequenzen definiert. Mit diesen Primern wurden PCR-Reaktionen durchgeführt. Die Amplifikate wurden mittels TOPO TA-Cloning (Invitrogen) in den bakteriellen Vektor pCR2.1 kloniert. Die Identität der Insertionen wurde durch Sequenzierung überprüft. Für alle Gene wurden Primer für die Amplifikation mit beidseitiger *Xma* I-Restriktionsschnittstelle definiert. Mit diesen Primern wurden PCR-Reaktionen durchgeführt. Als Template dienten die Konstrukte der Gene im Vektor pCR2.1. Die erhaltenen Fragmente wurden mit der Restriktionsendonuklease *Xma* I geschnitten. Der binäre Vektor pSS wurde ebenfalls mit *Xma I* geschnitten und mit alkaliner Phosphatase aus Kalbsthymus dephosphoryliert. Die geschnittenen Gene wurden in den Vektor pSS ligiert. Die erhaltenen Konstrukte wurden mittels Restriktionsanalysen hinsichtlich der Orientierung der Gene in *sense* oder *antisense* überprüft. Mehrere Klone wurden ausgewählt und zusätzlich durch Sequenzierung der Übergänge von pSS und Gen überprüft.
Die ausgewählten Konstrukte sowie der leere Vektor pSS (Kontrolle) wurden in kompetente S17.1-Zellen transformiert. Durch Konjugation wurden die Plasmide auf *Agrobacterium tumefaciens* pMP90RK übertragen. Die Agrobakterien-Kulturen wurden mittels PCR auf das Vorhandensein der Plasmide überprüft. Die Transformation von Tabakpflanzen mit den pSS-Konstrukten erfolgte über zwei unterschiedliche Ansätze. Ausgehend von 4 Wochen alten Sprosskulturen von *Nicotiana tabacum* SR1 wurden Protoplasten isoliert. Die Protoplasten wurden durch Cokultur mit den Agrobakterien transformiert. Dabei wurden die Agrobakterien mit den Konstrukten der Gene *in sense* und *antisense* für die Cokultur eingesetzt. Aus transformierten Protoplasten wurden unter entsprechendem Selektionsdruck Kalli gezogen. Je Konstrukt und Kontrolle wurden 70 Kalli regeneriert. Parallel wurden Blattscheiben-Transformationen mit allen Konstrukten durchgeführt.
Nach der Umsetzung auf kanamycinhaltigem Medium regenerierter Sprosse wurden die verbliebenen Kalli jedes einzelnen Konstrukts vereinigt. Von diesem Material wurde mRNA präpariert und als Slot-Blot auf Nitrocellulosemembran in 4 identischen Kopien übertragen. Je eine Kopie wurde mit je einer radioaktiven Sonde der PSY1, PDS, ZDS und LCY hybridisiert. In allen Proben zeigen sich starke Signale für die Transkripte der jeweils in die Pflanzen übertragenen Gene. Die Aktivität des doppelten 35S-Promoter ist damit nachgewiesen.
Die regenerierten Sprosse wurden in Sterilkultur bewurzelt. Die Spitze wurde auf neues Medium umgesetzt und der Spross heruntergeschnitten. Nach Bewurzelung der Spitze wurde der neu getriebene Spross aus der Sterilkultur im Gewächshaus in Erde gepflanzt. Die bewurzelte Spitze blieb in Sterilkultur und wurde in regelmäßigen Zeitabständen auf frisches Medium umgesetzt.
Von den transgenen Gewächshauspflanzen wurde nach 5-6 Wochen ein Blatt in einer Höhe von etwa $^2/_3$ der Pflanze abgeschnitten. Von diesem Blatt wurden 0,5 g mit einer Spatelspitze Magnesiumoxid und 3 ml Aceton im Potter zerkleinert. Der Aufschluss wurde filtriert und über HPLC der Gehalt an Xantophyllen, Carotinoiden und Chlorophyllen bestimmt. Das verbliebene Blattmaterial wurde sofort in flüssigem Stickstoff eingefroren und gemörsert. Von diesem Material wurden mRNA-Präparationen für Slot-Blots durchgeführt.

**Beispiel 6**

Phänotypische Analyse der transgenen Pflanzen

Die transgenen Pflanzen mit der Phytoene Synthase in *sense*-Orientierung zeigen sowohl in der Sterilkultur als auch im Gewächshaus phänotypisch zum Teil starke Effekte. Die Blattmorphologie ist stark beeinflusst: junge Blätter weisen zum Teil eine leicht orange-gelbe Färbung auf, die jedoch mit zunehmendem Alter verblasst, ältere Blätter weisen dann eine sehr unregelmäßige blassgrüne Pigmentierung auf. Die Blätter sind zudem dickfleischig, behaart und sehr fest, ihre Ränder rollen sich von den Seiten nach innen. Die Pflanzen sind insgesamt in ihrer Entwicklung gegenüber Kontrollpflanzen stark retardiert. Die Blüten einiger besonders stark betroffener Pflanzen sind fast farblos und weisen nicht die den Kontrollpflanzen eigene rosa Färbung der Blütenblätter auf. Die Samenkapseln sind deutlich orange statt grün gefärbt. Die Messung der Carotinoid-, Xanthophyll- und Chlorophyllgehalte dieser Pflanzen zeigt die Akkumulation geringer Mengen von Phytoen und einen deutlichen Anstieg des β-Carotin-Gehaltes. Phytoen als direktes Produkt der Phytoene Synthase lässt sich in Kontrollpflanzen unter den gegebenen Bedingungen nicht nachweisen. Des weiteren zeigen die transgenen Pflanzen eine Reduktion im Chlorophyllgehalt von bis zu 40%. Dies lässt sich auf die Umleitung von Geranylgeranylpyrophosphat aus der Phytolsynthese in die Carotinoidsynthese zurückführen, die dann in Folge zu einer reduzierten Chlorophyllsynthese führt.

Transgene Pflanzen mit der Phytoene Desaturase in *antisense*-Orientierung zeigen im Gewächshaus Effekte in der Blattpigmentierung. Die Blätter weisen weiße Adern auf, die Blattspitzen sind teilweise vollständig weiß. Die Samenkapseln einer Linie waren ebenfalls vollständig weiß. Die Messung der Carotinoidgehalte der betroffenen Linien zeigt eine sehr starke Akkumulation von Phytoen, die ungefähr in der Größenordnung liegt, die bei Norflurazon-behandelten Tabakpflanzen gefunden wird. Die Gehalte an Xanthophyllen und Chlorophyll sind reduziert. Von einer der betroffenen Linien wurden Samen einer geselbsteten Pflanze ausgelegt und unter Selektion zur Keimung gebracht. Diese Keimlinge zeigten eine Aufspaltung in 3 Phänotypen. Selektionierten Pflanzen, die nur weiße Cotyledonen aufwiesen, sowie grüne und weiße Pflanzen, die über die Primärblätter hinauswuchsen. Bei den weißen Keimlingen handelt es sich um homozygot transgene Pflanzen. Nach Umsetzen in das Gewächhaus starben alle weißen Pflanzen innerhalb einer Woche, die grünen zeigen den beschriebenen Phänotyp der adulten heterozygoten Pflanzen. Die Bestimmung der Profile zeigte für die grünen Keimlinge eine sehr starke Akkumulation von Phytoen. Bei den weißen Keimlingen konnte kein β-Carotin und nur noch geringe Mengen an Xanthophyllen nachgewiesen werden.

**Literatur**

Altschul, S.F., Madden, T.L., Schaffer, A.A., Zhang, J.Z.; Miller W. and Lipman, D.J. 1997. Gapped BLAST und PSI-BLAST generation of protein database search programs. Nucleic Acids Res. 25: 3389-3402

Lichtenthaler, H.K., Schwender, J., Disch, A., Rohmer, M. Biosynthesis of isoprenoids in higher plant chloroplasts proceeds via a mevalonate-independent pathway. FEBS Letters (1997) 400, 271-274.

Pecker, I., Chamovitz, D., Linden, H., Sandmann, G., Hirschberg, J. A single polypeptide catalyzing the conversion of phytoene to zeta-carotene is transriptionally regulated during tomato fruit ripening. Plant Molecular Biology (1996) 30, 807-819.

Bonk, M., Hoffmann, B., Lintig, von, J., Schledz, M., Al-Babili, S., Hobeika, E., Kleinig, H., Beyer, P. Chloroplast import of four carotenoid biosynthetic enzymes *in vitro* reveals differetial fates prior to membrane binding and oligomeric assembly. Eur. J. Biochem. (1997) 247, 942-950.

Dogbo, O., Laferrière, A., d'Harlingue, A., Camara, B. Carotenoid biosynthesis: Isolation and characterization of a bifunctional enzyme catalyzing the synthesis of phytoene. PNAS (1988) 85, 7054-7058.

Fire, A., Xu, S. Montgomery, M.K., Kostas, S.A., Driver, S.E., Mello, C.C. Potent and specific genetic interference by double-stranded RNA in *Caenorhabditis elegans*. Nature (1998) 391, 806-811.

Fray, R.G., Wallace, A., Fraser, P.D., Valero, D., Hedden, P., Bramley, P.M., Grierson, D. Constitutive expression of a fruit phytoene synthase gene in transgenic tomatoes causes dwarfism by redirecting metabolites from the gibberellin pathway. Plant J. (1995) 8 (5), 693-701.

Lintig, von , J, Welsch, R., Bonk, M., Giuliano, G., Batschauer, A., Kleinig, H. Light-dependant regulation of carotenoid biosynthesis occurs at the level of phytoene synthase exoression and is mediated by phytochrome in *Sinapis alba* and *Arabidopsis thaliana* seedligs. Plant J. (1997) 12(3), 625-634.

Lottspeich, F., Zorbas H. (Hrsg.) 1998. Bioanalytik. Spektrum Akademischer Verlag, Heidelberg, Berlin.

Minet, M., Dufour, M.-E. and Lacroute, F. 1992. Complementation of *Saccharomyces cerevisiae* auxotrophic mutants by *Arabidopsis thaliana* cDNAs. Plant J. 2: 417-422.

Neudert, U., Martinez, I.M., Fraser, P.D., Sandmann, G. Expression of an active phytoene synthase form *Erwinia*

*uredovora* and biochemical properties of the enzyme. Biochimics et Biophysica Acta (1998) 1392, 51-58.

Norris, S.R., Barrette, T., DellaPenna, D. Genetic dissection of carotenoid synthesis in Arabidopsis defines plastoquinone as an essential component of phytoene desaturation. Plant Cell (1995) 7, 2139-2149.

Wetzel, C.M., Rodermel, S.R. Regulation of phytoene desaturase expression is independant of leaf pigment content in *Arabidopsis thaliana*. Plant Molecular Biology (1998) 37, 1045-1053.

Corona, V., Aracri, B., Kosturkova, G., Bartley, G.E., Pitto, L., Giorgetti; L. Scolnik, P.A.., Giuliano, G. Regulation of a carotenoid biosynthesis gene promoter during plant development. The Plant J. (1996) 9(4), 505-512.

Trebst, A., Depka, B. Role of carotene in the rapid turnover and assembly of photosystem II in *Chlamydomonas reinhardtii*. FEBS Letters (1997) 400, 359-362.

Cunningham, F.X., Pogson, B., Sun, Z., McDonald, K.A., DellaPenna, D., Gantt, E. Functional analysis of the β and ε lycopene cyclase enzymes of Arabidopsis reveals a mechanism for control of cyclic carotenoid formation. Plant Cell, 8: 1613-1626, 1996.

**Erläuterungen zum Sequenzprotokoll und zu den Abbildungen**

**SEQ ID NO. 1**

DNA-Sequenz kodierend für eine Phytoene Synthase aus *Nicotiana tabacum*. Die von der DNA kodierte Aminosäuresequenz ist angegeben.

**SEQ ID NO. 2**

Aminosäuresequenz eines Polypeptids mit der Aktivität einer Phytoene Synthase aus *Nicotiana tabacum*.

**SEQ ID NO. 3**

DNA-Sequenz kodierend für eine Phytoene Synthase aus *Nicotiana tabacum*. Die von der DNA kodierte Aminosäuresequenz ist angegeben.

**SEQ ID NO. 4**

Aminosäuresequenz eines Polypeptids mit der Aktivität einer Phytoene Synthase aus *Nicotiana tabacum*.

**SEQ ID NO: 5**

DNA-Sequenz kodierend für die Zeta Carotene Desaturase aus *Nicotiana tabacum*. Die von der DNA kodierte Aminosäuresequenz ist angegeben.

**SEQ ID NO: 6**

Aminosäuresequenz eines Polypeptids mit der Aktivität einer Zeta Carotene Desaturase aus *Nicotiana tabacum*.

**SEQ ID NO: 7**

Oligonukleotid zur Amplifikation der Zeta-Carotene Desaturase aus *Nicotiana tabacum* mittels PCR-Technik.

**SEQ ID NO: 8**

Oligonukleotid zur Amplifikation der Zeta-Carotene Desaturase aus *Nicotiana tabacum* mittels PCR-Technik.

**SEQ ID NO: 9**

Oligonukleotid zur Amplifikation der Phytoene Desaturase aus *Nicotiana tabacum* mittels PCR-Technik.

**SEQ ID NO: 10**

Oligonukleotid zur Amplifikation der Phytoene Desaturase aus *Nicotiana tabacum* mittels PCR-Technik.

**Abbildung 1**

Carotinoidbiosynthese in den Plastiden von Pflanzen; Abkürzungen: PSY: Phytoene Synthase; PDS: Phytoene Desaturase; ZDS: Zetacarotene Desaturase; LCY: Lycopene β-Cyclase.

**Abbildung 2**

Überprüfung der Transkriptakkumulation in transgenen Kalli: Nach Umsetzung regenerierter Sprosse wurden die verbliebenen Kalli einer Transformation vereinigt. Von diesen Kalli-Mischungen wurde mRNA präpariert. Je ~500 ng der mRNA wurde auf Nitrocellulosemembran entsprechend dargestelltem Probenschema geblottet. Je eine der Membranen wurde mit einer radioaktiven Sonde der vier verschiedenen Gene hybridisiert. Als Kontrollen wurde mRNA von 4 Wochen alten Tabak-Keimlingen und von 4 Wochen alten Reis-Keimlingen verwendet. Abkürzungen: Pp: Protoplasten, K3 o,4M: K3 0,4M-Medium, KPS:KPS-Medium.

**Patentansprüche**

1.  Nukleinsäuren, die für Polypeptide aus Tabak mit der biologischen Aktivität einer Zeta Carotene Desaturase, welche die Aminosäuresequenz gemäß SEQ ID NO: 6 umfasst, kodieren.

2.  Nukleinsäuren gemäß Anspruch 1, **dadurch gekennzeichnet, dass** sie für Polypeptide mit der Aminosäuresequenz gemäß SEQ ID NO: 6 kodieren.

3.  Nukleinsäuren gemäß Anspruch 1 oder 2, **dadurch gekennzeichnet, dass** es sich um einzelsträngige oder doppelsträngige DNA oder RNA handelt.

**4.** Nukleinsäuren gemäß Anspruch 3, **dadurch gekennzeichnet, dass** es sich um Fragmente genomischer DNA oder cDNA handelt.

**5.** Nukleinsäuren gemäß einem der Ansprüche 1 bis 4, **dadurch gekennzeichnet, dass** sie aus Tabakpflanzen stammen.

**6.** Nukleinsäuren gemäß einem der Ansprüche 1 bis 5, umfassend eine Sequenz ausgewählt aus

(a) der Sequenz gemäß SEQ ID NO: 5

(b) Sequenzen, die für ein Polypeptid kodieren, welches die Aminosäuresequenz gemäß SEQ ID NO: 6 umfasst,

(c) zumindest 14 Basenpaare langen Teilsequenzen der unter (a) oder (b) definierten Sequenzen,

(d) Sequenzen, welche an die unter (a), (b) oder (c) definierten Sequenzen hybridisieren,

(e) Sequenzen, welche zu den unter (a), (b) oder c) definierten Sequenzen komplementär sind, und

(f) Sequenzen, welche aufgrund der Degeneriertheit des genetischen Codes für dieselbe Aminosäuresequenz kodieren wie die unter a) bis c) definierten Sequenzen.

**7.** Regulatorische Region, welche natürlicherweise die Transkription einer Nukleinsäure gemäß einem der Ansprüche 1 bis 6 in Pflanzenzellen, insbesondere in Tabakpflanzen, kontrolliert.

**8.** DNA-Konstrukt umfassend eine Nukleinsäure gemäß einem der Ansprüche 1 bis 6 und einen heterologen Promotor.

**9.** Vektor umfassend eine Nukleinsäure gemäß einem der Ansprüche 1 bis 6, eine regulatorische Region gemäß Anspruch 7 oder ein DNA-Konstrukt gemäß Anspruch 8.

**10.** Vektor gemäß Anspruch 9, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

**11.** Wirtszelle enthaltend eine Nukleinsäure gemäß einem der Ansprüche 1 bis 6, ein DNA-Konstrukt gemäß Anspruch 8 oder einen Vektor gemäß Anspruch 9 oder 10.

**12.** Wirtszelle gemäß Anspruch 11, **dadurch gekennzeichnet, dass** es sich um eine prokaryotische Zelle, insbesondere um *E. coli*, handelt.

**13.** Wirtszelle gemäß Anspruch 11, **dadurch gekennzeichnet, dass** es sich um eine eukaryotische Zelle, insbesondere um eine Hefe-, Insekten-, Säugetier oder Pflanzenzelle, handelt.

**14.** Polypeptid mit der biologischen Aktivität einer Phytoene Synthase, welches von einer Nukleinsäure gemäß SEQ ID NO:1 oder SEQ ID NO:3 kodiert wird, umfassend eine Aminosäuresequenz gemäß SEQ ID NO:2 oder SEQ ID NO:4.

**15.** Polypeptid mit der biologischen Aktivität eine Zeta Carotene Desaturase, welches von einer Nukleinsäure gemäß einem der Ansprüche 1 bis 6 kodiert wird.

**16.** Antikörper, welcher spezifisch an ein Polypeptid gemäß Anspruch 14 bindet.

**17.** Antikörper, welcher spezifisch an ein Polypeptid gemäß Anspruch 15 bindet.

**18.** Verfahren zum Herstellen einer Nukleinsäure gemäß einem der Ansprüche 1 bis 6, umfassend die folgenden Schritte:

(a) Vollständige chemische Synthese auf an sich bekannte Weise oder

(b) chemische Synthese von Oligonukleotiden, Markieren der Oligonukleotide, Hybridisieren der Oligonukleotide an DNA einer genomischen oder cDNA-Bank, die ausgehend von genomischer DNA bzw. mRNA aus Pflanzenzellen hergestellt wurde, Selektieren von positiven Klonen und Isolieren der hybridisierenden DNA aus positiven Klonen oder

(c) chemische Synthese von Oligonukleotiden und Amplifizierung der Ziel-DNA mittels PCR.

**19.** Verfahren zum Herstellen eines Polypeptids gemäß Anspruch 14 oder 15, umfassend

(a) das Kultivieren einer Wirtszelle gemäß einem der Ansprüche 11 bis 13 unter Bedingungen, die die Expression der Nukleinsäure gemäß einem der Ansprüche 1 bis 6 gewährleisten, oder

(b) das Exprimieren einer Nukleinsäure gemäß einem der Ansprüche 1 bis 6 in einem *in vitro-System,* und

(c) die Gewinnung des Polypeptids aus der Zelle, dem Kulturmedium oder dem *in vitro*-System.

**20.** Verfahren zum Auffinden einer chemischen Verbindung, die an ein Polypeptid gemäß Anspruch 14 und/oder 15 oder ein Polypeptid mit der biologischen Aktivität einer Phytoene Desaturase bindet, umfassend die folgenden Schritte:

(a) Inkontaktbringen einer Wirtszelle gemäß einem der Ansprüche 11 bis 13, eines Polypeptids gemäß Anspruch 14 oder 15 oder eines Polypeptids mit der biologischen Aktivität einer Phytoene Desaturase mit einer chemischen Verbindung oder einem Gemisch von chemischen Verbindungen unter Bedingungen, die die Interaktion einer chemischen Verbindung mit dem Polypeptid erlauben, und

(b) Bestimmen der chemischen Verbindung, die spezifisch an das Polypeptid bindet.

**21.** Verfahren zum Auffinden einer Verbindung, welche die Expression von Polypeptiden gemäß Anspruch 14, 15 oder eines Polypeptids mit der biologischen Aktivität einer Phytoene Desaturase verändert, umfassend die folgenden Schritte:

(a) Inkontaktbringen einer Wirtszelle gemäß einem der Ansprüche 11 bis 13 mit einer chemischen Verbindung oder einem Gemisch von chemischen Verbindungen,

(b) Bestimmen der Polypeptidkonzentration, und

(c) Bestimmen der Verbindung, welche die Expression des Polypeptids spezifisch beeinflusst.

**22.** Verwendung einer Nukleinsäure gemäß einem der Ansprüche 1 bis 6, eines DNA-Konstrukts gemäß Anspruch 8, eines Vektors gemäß Anspruch 9 oder 10, einer Wirtszelle gemäß einem der Ansprüche 11 bis 13, eines Polypeptids gemäß Anspruch 14 oder 15 oder eines Polypeptids mit der biologischen Aktivität einer Phytoene Desaturase oder eines Antikörpers gemäß Anspruch 16 oder 17 zum Auffinden neuer herbizider Wirkstoffe.

**23.** Verwendung eines Modulators eines Polypeptids gemäß Anspruch 14 oder 15 oder eines Polypeptids mit der biologischen Aktivität einer Phytoene Desaturase als Pflanzenwuchsregulator oder Herbizid.

**24.** Verwendung einer Nukleinsäure gemäß einem der Ansprüche 1 bis 6, eines DNA-Konstrukts gemäß Anspruch 8 oder eines Vektors gemäß Anspruch 9 oder 10 zum Herstellen von transgenen Pflanzen.

**25.** Transgene Pflanzen, Pflanzenteile, Protoplasten, Pflanzengewebe oder Pflanzenvermehrungsmaterialien, **dadurch gekennzeichnet, dass** nach Einbringen einer Nukleinsäure gemäß einem der Ansprüche 1 bis 6, eines DNA-Konstrukts gemäß Anspruch 8 oder eines Vektors gemäß Anspruch 9 die intrazelluläre Konzentration eines Polypeptids gemäß Anspruch 16 oder 17 im Vergleich zu den entsprechenden Wildtyp-Zellen erhöht oder vermindert ist.

**26.** Pflanzen, Pflanzenteile, Protoplasten, Pflanzengewebe oder Pflanzenvermehrungsmaterialien, **dadurch gekennzeichnet, dass** sie ein Polypeptid gemäß Anspruch 14 oder 15 enthalten, dessen biologische Aktivität oder Expressionsmuster im Vergleich zu den entsprechenden endogenen Polypeptiden verändert ist.

27. Verfahren zum Herstellen von Pflanzen, Pflanzenteilen, Protoplasten, Pflanzengeweben oder Pflanzenvermehrungsmaterialien gemäß Anspruch 25, **dadurch gekennzeichnet, dass** man eine Nukleinsäure gemäß einem der Ansprüche 1 bis 6 oder eine regulatorische Region gemäß Anspruch 7 durch endogene Mutagenese verändert.

**Abbildung 1**

**Probenschema der 4 identischen Blots:**

| SRI Kontrolle (+KO) | O.sativa Kontrolle (-KO) | | | | | | |
|---|---|---|---|---|---|---|---|
| Pp K3Q4M t=0h | Pp K3Q4M t=2h | Pp K3Q4M t=4h | Pp KPS t=0h | Pp KPS t=2h | Pp KPS t=4h | | |
| Pool PSY antisense | Pool PSY sense | Pool PDS antisense | Pool PDS sense | Pool ZDS antisense | Pool ZDS sense | Pool LCY antisense | Pool LCY sense |
| Pool PSY antis./sens | Pool PDS antis./sens | Pool ZDS antis./sens | Pool LCY antis./sens | Pool pSS | | | |

▉ : Kontrolle *N. tabacum* SR1, 4 Wochen alte Keimlinge

▢ : Kontrolle *O.sativa*, 4 Wochen alte Keimlinge

⋮ :bei Transkription des Transgens durch 35S-Promoter erwartetes starkes Signal

**Abbildung 2**